# EUROPEAN PATENT SPECIFICATION

(11) **EP 3 812 472 B1**
(45) Date of publication and mention of the grant of the patent: **23.11.2022**
(21) Application number: 19204418.8
(22) Date of filing: 21.10.2019
(51) Int. Cl.: C12Q 1/6855

(54) **A TRULY UNBIASED IN VITRO ASSAY TO PROFILE OFF-TARGET ACTIVITY OF ONE OR MORE TARGET-SPECIFIC PROGRAMMABLE NUCLEASES IN CELLS (ABNOBA-SEQ)**
IN-VITRO-TEST ZUM PROFILIEREN DER OFF-TARGET-AKTIVITÄT EINES ODER MEHRERER ZIELSPEZIFISCHER PROGRAMMIERBARER NUKLEASEN IN ZELLEN (ABNOBA-SEQ)
DOSAGE IN VITRO VRAIMENT NON BIAISÉ POUR PROFILER UNE ACTIVITÉ HORS CIBLE D'UNE OU DE PLUSIEURS NUCLÉASES PROGRAMMABLES SPÉCIFIQUES À UNE CIBLE DANS DES CELLULES (ABNOBA-SEQ)

(43) Date of publication of application: 28.04.2021
(73) Proprietor: Albert-Ludwigs-Universität Freiburg, 79098 Freiburg (DE)
(72) Inventor: CATHOMEN, Toni, 79106 Freiburg (DE); HAAS, Simone Alexandra, 79115 Freiburg (DE); HILDENBEUTEL, Markus, 69469 Weinheim (DE); MUSSOLINO, Claudio, 79115 Freiburg (DE); BÖRRIES, Melanie, 79299 Wittnau (DE); ANDRIEUX, Geoffroy, 68180 Horbourg-Wihr (FR)
(74) Representative: Lederer & Keller Patentanwälte Partnerschaft mbB

(56) References cited:
- WO-A1-2016/081798
- WO-A1-2018/013558
- HAAS: "ESGCT XXV Anniversary Congress in Collaboration with the German Society for Gene Therapy October 17-20, 2017 Berlin, Germany", HUMAN GENE THERAPY, vol. 28, no. 12, 1 December 2017 (2017-12-01), pages A1-A125, XP55688705, GB ISSN: 1043-0342, DOI: 10.1089/hum.2017.29055.abstracts
- Beeke Wienert ET AL: "Abstract", bioRxiv, 14 November 2018 (2018-11-14), XP055688684, DOI: 10.1101/469635 Retrieved from the Internet: URL:https://science.sciencemag.org/content /364/6437/286.full.pdf
- LEE MINYOUNG ET AL: "Therapeutic application of the CRISPR system: current issues and new prospects", HUMAN GENETICS, SPRINGER, BERLIN, DE, vol. 138, no. 6, 21 May 2019 (2019-05-21), pages 563-590, XP036960291, ISSN: 0340-6717, DOI: 10.1007/S00439-019-02028-2 [retrieved on 2019-05-21]

## Description

### FIELD OF THE INVENTION

The present invention relates to a method for detecting off-target sites of one or more target-specific programmable nucleases in a genome *in vitro,* allowing the detection of off-target sites in an input sample of less than 1 µg of genomic DNA. The present invention also relates to a method for detecting *in vivo* off-target sites by using the *in vitro* method for detecting off-target sites of one or more target-specific programmable nucleases in a genome.

### BACKGROUND

Genome editing with nucleases of the Clustered Regularly Interspaced Short Palindromic Repeats (CRISPR)/Cas type, but also Transcription Activator-Like Effector Nucleases (TALEN) and Zinc Finger Nucleases (ZFN), is already being used therapeutically in clinical studies. These custom-made target-specific programmable nucleases can be targeted to a specific region in the genome, where they introduce a double-stranded DNA (dsDNA) break, which triggers cellular DNA repair mechanisms. The introduced dsDNA breaks are repaired by either of two major cellular repair pathways, non-homologous end joining (NHEJ) or homology-directed repair (HDR) (Hustedt and Durocher, 2016, Nat Cell Biol 19(1): 1-9).

While target-specific programmable nucleases like ZFN, TALEN and meganucleases rely on protein-DNA recognition, the specificity of CRISPR/Cas RNA-guided nucleases (CRISPR/Cas RGN) depends on RNA-DNA recognition. In CRISPR/Cas RGN a guide RNA (gRNA) hybridizes with an approximately 20 nucleotide long sequence (target sequence) next to a protospacer-adjacent motif (PAM) and enables the Cas endonuclease to cleave the target site (Wiedenheft et al., 2012, Nature 482, 331-338; Terns et al., 2011, Curr Opin Microbiol 14, 321-327). Changing the nucleotide sequence of the 5' end of the gRNA modifies the site targeted by the CRISPR/Cas RGN. In its active form the CRISPR/Cas RGN is a ribonucleoprotein (RNP), wherein Cas endonuclease is bound to a guide RNA (gRNA). Commonly used Cas endonucleases include Cas9, Cpf1 and variants thereof. For example, different Cas9 endonucleases derived from *Streptococcus pyogenes, Neisseria meningitidis, Staphylococcus aureus, Streptococcus thermophilus,* and *Treponema denticola* have been used to develop genome editing tools.

In addition to the actual target sequence (on-target site), target-specific programmable nucleases can be active at sites in the genome that differ in sequence to some degree from the actual target sequence, the so-called off-target sites, thereby generating dsDNA break mutations at off-target sites that differ by up to several nucleotides from on-target sites. For example, it has been shown that CRISPR-Cas9 nucleases can tolerate up to six mismatches between the gRNA and the target DNA, especially in the PAM-distal region of the target site (e.g. Tsai et al., 2015, Nat Biotechnol 33(2): 187-197).

Off-target activity in human cells can have potentially perilous consequences. The generation of dsDNA breaks in off-target sites in the genome can produce insertions and deletions (indels) as well as translocations that need to be carefully monitored. If an off-target site is for examples located in a coding region or in a regulatory element, in the worst case a carcinogenic degeneration of the cell can be induced. Therefore, the off-target activity of target-specific programmable nucleases must be determined and, if necessary, the specificity of the target-specific programmable nucleases used must be optimized before the genome editing tools can be used in clinical settings. Thus, the understanding and preventing of off-target effects is critical in context of the therapeutic use of target-specific programmable nucleases.

Importantly, individual differences in the genome, such as single-nucleotide polymorphisms (SNPs), as well as the cell-type specific chromatin status, can change the on- and off-target profiles of any target-specific programmable nuclease (Lessard et al., 2017, Proc Natl Acad Sci U S A 114(52): E11257-E11266, Verkuijl and Rots, 2019, Curr Opin Biotechnol 55: 68-73.). This further underlines the need to identify potential off-target sites on a cell-type specific and individualised basis before the therapeutic application of a particular target-specific programmable nuclease.

Different methods, with corresponding advantages and disadvantages, have been developed to determine the off-target activity of target-specific programmable nucleases (summarized in Kim et al., 2019, Annu Rev Biochem 88: 191-220). On the one hand, potential off-target sites can be predicted *in silico* using bioinformatic algorithms on the basis of sequence similarity of such potential off-target sites to the actual target sequence and may then be investigated by target amplicon sequencing for example using next generation sequencing (NGS). The effects of already a single mismatch compared to the on-target sequence are not always predictable. Additionally, the genomic or epigenomic context, or both, might also affect the cleavage frequency. These factors make it very difficult to develop an algorithm capable of identifying potential off-target sites. Such *in silico* based methods are fast and relatively inexpensive. However, these *in silico* methods tend to have a low true positive rate (low sensitivity). Off-target activity at sites overlooked by such algorithms is not detected.

As an alternative, off-target activity can be determined experimentally, which means the detection is not initiated by computer-aided predictions. Experimental methods are more labour-intensive and expensive. They are also subject to technical and bioinformatic limitations that need to be overcome. Different experimental approaches for determining specificity are known, for example GUIDE-Seq, DISCOVER-Seq, Digenome-Seq, CIRCLE-Seq and SITE-Seq. They can be roughly divided into cell-based methods and *in vitro* methods.

US 2018/0016572 discloses an optimized version of GUIDE-seq and stands for Target-Enriched GUIDE-seq which is also designated short as TEG-seq. The method is based on detecting and mapping of double stranded short DNA tags that are integrated into the DNA double strand breaks generated by a CRISPR-Cas nuclease, whereby the method is cell-based that detects off-target activity in cell lines.

Wienert et al. "Unbiased detection of CRISPR off-targets in vivo using DISCOVER-Seq,", Science (2019), 364, pp 286-289 disclose the so-called DISCOVER-Seq which is a method to detect off-target activity of CRISPR-Cas nucleases. It is a method that uses cell lines such as human K562 cells or murine B16-F10 cells. The method is based on detecting off-target activity in cells by using ChIP-Seq. It exhibits both low specificity and low sensitivity. Since the method requires for example 2-10 million cells as experimental input, this method is poorly suited for an individualized analysis of the specificity of target-specific programmable nucleases, in particular of cells with a limited sample size and limited availability.

WO 2016/081798 disclose methods relating to the detection of recurrent and non-specific double strand breaks in the genome. The method is cell-based and the method should detect translocations. This method is not very sensitive since it requires 20-100 µg of genomic input DNA which corresponds approximately to an equivalent of 3-15 million cells.

The cell-based assays GUIDE-seq (Tsai et al., 2015, Nat Biotechnol 33(2): 187-197) and DISCOVER-seq (Wienert et al., 2019, Science 364(6437): 286-289) tag off-target sites in living cells by harnessing the cellular repair mechanisms that are triggered by target-specific programmable nuclease activity. GUIDE-Seq is a cell-based method in which short, double-stranded oligodesoxynucleotides (dsODN) are introduced into the cell in addition to a target-specific programmable nuclease. If the nuclease cleaves the genome, short dsODN are integrated into the DNA at the site of double strand breaks, which then serve as the starting point for high-throughput sequencing. This method works well, but only in certain cell lines that can differ from cells originating from different individual at key sites in the genome.

DISCOVER-Seq is another cell-based method, which is based on the ChIP-seq method. If the genetic material is cut by target-specific programmable nucleases, this triggers cellular repair mechanisms and DNA repair proteins, such as MRE11, are recruited to the DNA double-strand break. DNA is then cross-linked/fixed with the proteins bound to it. The resulting "chromatin DNA" is fragmented and all DNA fragments with bound MRE11 protein are precipitated using an antibody directed against MRE11. Finally, by NGS of the isolated DNA fragments, the sites with off-target activity can be identified. This method measures actual dsDNA breaks in the cell, however also those not induced by target-specific programmable nucleases. Moreover, this method can only detect DNA double-strand breaks that were not yet repaired at the time of cell fixation. It can therefore be assumed that both the number of false-positive and the small number of correctly-positive results limit the applicability of this method.

In general, these cell-based methods work very well in certain cell lines. Due to the observed toxicity of dsODN for example in primary cells, however, GUIDE-Seq cannot be easily or not at all transferred to certain cells, whereas DISCOVER-Seq will exhibit both low specificity and sensitivity. Therefore, these methods are poorly suited for an individualized analysis of the specificity of target-specific programmable nucleases, in particular of cells with a limited sample size and availability.

The *in vitro* methods Digenome-Seq (Kim et al, 2015, Nat Methods 12(3): 237-243), CIRCLE-Seq (Tsai et al., 2017, Nat Methods 14(6): 607-614) and SITE-Seq (Cameron et al., 2017, Nat Methods 14(6): 600-606) uncover off-target sites by tagging the sites that are cleaved in isolated genomic DNA by CRISPR-Cas RGN *in vitro.*

In Digenome-Seq, the genomic DNA is cleaved in the test tube using target-specific programmable nucleases. DNA adapters are ligated to the fracture sites, to which primers can bind, which are then used for high-throughput sequencing. A major disadvantage of this approach is that a reference genome must also be sequenced and that, due to the lack of an enrichment step, sequencing can only be carried out on expensive high-performance sequencers (e.g. HiSeq), which makes this approach very expensive. Moreover, relatively large amounts of genomic input DNA have to be used. This method corresponds to the subject matter of the European patent application published as EP 3 219 810 A1.

In CIRCLE-Seq, the genomic DNA is first fragmented by ultrasound and the individual pieces are then ligated into ring-shaped molecules. In a next step, the circular DNA is cut with CRISPR-Cas RGN, resulting in linearized molecules. To the ends of these linearized molecules adapters are ligated, which can then be used for sequencing and identification of the off-target sites. The main disadvantage of this method is that large amounts of genomic input DNA have to be used. This method corresponds to the subject matter of the U.S. patent with the patent no. US 9,850,484.

In SITE-Seq, the genomic DNA is cleaved in the test tube using target-specific programmable nucleases. Biotinylated adapters are ligated to the cleaved sites, through which cleaved molecules can be enriched after enzymatic fragmentation. Then, adapters are ligated at their ends, which can be used for sequencing and identification of the off-targets. Also this method requires relatively large amounts of genomic input DNA.

A main disadvantage of these *in vitro* methods is that relatively large amounts of genomic input DNA have to be used (about 8 to 25 µg depending on the method), which makes off-target analysis in valuable cell samples of limited sample size and availability difficult or even impossible. Moreover, *in vitro* methods are generally characterized by a high false positive rate (low specificity), i.e. many of the off-target sites cut *in vitro* are not cut in the cell, for example because the concentration of target-specific programmable nucleases in the cell is lower or because these sites are not accessible due to the chromatin configuration (Kim et al., 2019, Annu Rev Biochem 88: 191-220).

To summarize, the use of the above methods in an individualized way on cells with a limited sample size, is mainly limited due to their requirement of a high amount of input DNA of the current *in vitro* assays or the fact that the cell-based methods are not easily transferrable to certain cells, e.g. primary cells.

There exists a need for a method for detecting off-target sites of one or more target-specific programmable nucleases in a genome that allows for detection in an individualized manner of off-target sites in genomic DNA of precious cell samples with a limited sample size and availability.

### SUMMARY OF THE INVENTION

The present inventors have found a method for detecting off-target sites of one or more target-specific programmable nucleases in a genome *in vitro* (Abnoba-Seq), which allows for detection of off-target sites in the field of genome editing in precious cell samples of limited sample size. In contrast to established *in vitro* methods like Digenome-Seq, CIRCLE-Seq and SITE-Seq, the inventive method requires only minimal amounts of genomic input DNA (< 1 µg), allowing for testing of one or more target-specific programmable nucleases on very small cell samples of limited supply, for example on the genomic DNA derived from primary cells, biopsy material or a gene-edited graft. Moreover, the inventive method can be carried out with basic (low-cost) NGS sequencers, requiring no expensive high-performance sequencer like the HiSeq Sequencing Systems. Finally, the method can identify off-target sites with comparable sensitivity as already established *in vitro* methods, but with a lower false positive rate, which means with a significantly better specificity than comparable methods.

In a second aspect the present invention, furthermore, provides a method for detecting *in vivo* off-target sites by using the method for detecting off-target sites of one or more target-specific programmable nucleases in a genome *in vitro* (Dual-Abnoba-Seq). This method thus allows for a determination of *in vivo* specificity of one or more target-specific programmable nucleases in precious cell samples of limited sample size and availability, in other words the ability to determine actual off-target activity of the one or more target-specific programmable nucleases in specific cells of small sample size. This means that the method according to the second aspect of the present invention can be used to test target-specific programmable nuclease activity *in vivo* to demonstrate the actual *in vivo* activity of one or more specific target-specific programmable nucleases in a particular tissue or cell type of limited sample size and availability.

### BRIEF DESCRIPTION OF THE DRAWINGS

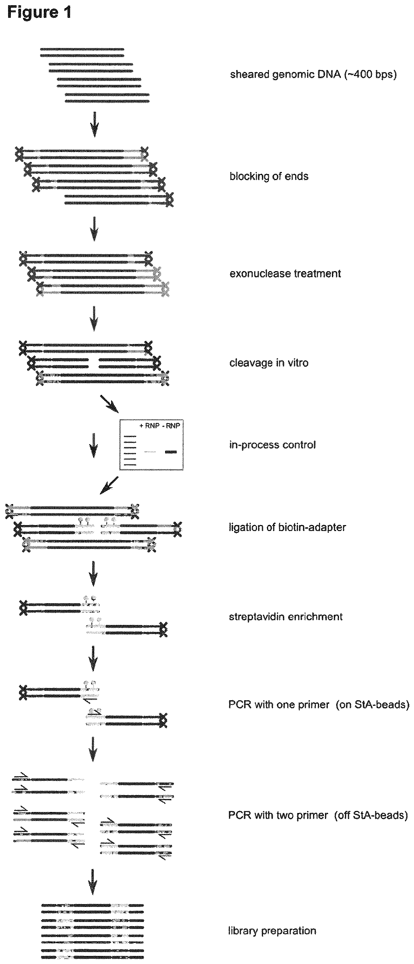

*Figure 1**: Overview of Abnoba-Seq workflow*
   Genomic DNA was randomly sheared to a median fragment size of ~ 400 bp, ends protected by ligation of a blocking adapter (gray), and non-protected fragments removed by exonuclease treatment. End-blocked DNA fragments were then cleaved with CRISPR-Cas9, and successful cleavage of the genome controlled by PCR amplification of the on-target site as an in-process control (IPC). Cleaved fragments were tagged by ligation of a biotin-adapter (light gray), enriched on streptavidin (StA) beads, and subsequently amplified by PCR: first, a linear PCR on the StA beads with a single primer binding to the biotin-adapter, and second by exponential PCR after the addition of a second primer that binds to the blocking-adapter. Finally, a library was prepared and subjected to next generation sequencing (NGS).

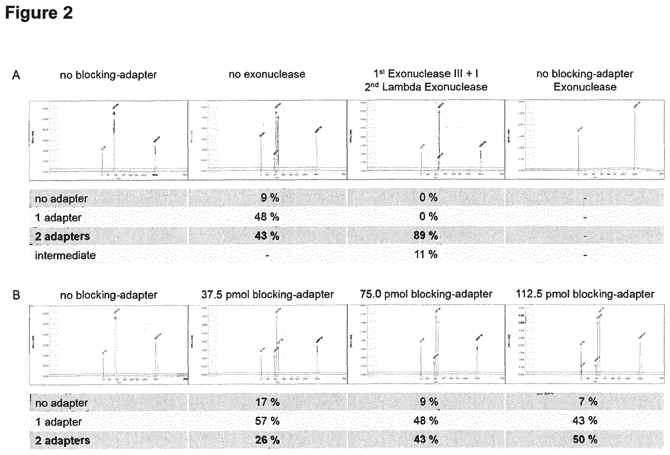

*Figure 2**: Testing end-blocking of DNA fragments*
   Ligation of blocking-adapter to DNA fragments (PCR amplicons), followed by an exonuclease treatment and subsequent capillary electrophoresis is shown on top, with the percentages of end-blocked DNA fragments (PCR amplicons) in the tables below the graphs. A: Exonuclease treatment: Blocking-adapter (75 pmol) ligation to a 189 bp PCR amplicon (400 ng) and subsequent digestion with a combination of exonucleases for 30 min in a two-step reaction with Exonuclease III (200 U), Lambda Exonuclease (10 U) and Exonuclease I (20 U). B: Titration of blocking-adapter concentration: Ligation of blocking-adapter in different concentrations to a 189 bp PCR amplicon (400 ng). The PCR amplicon alone, with blocking-adapter ligated to one end or with blocking-adapter ligated to both ends is represented by the 189 bp peak, the 215 bp peak or the 241 bp peak, respectively, and an intermediate by the 225 bp peak. 15 bp and 5,000 bp peaks (first and last peak), fragments of alignment marker; percentages were calculated by means of AUC.

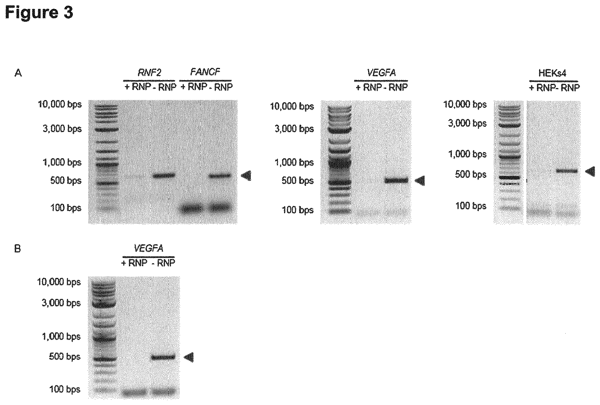

*Figure 3**: In-process controls*
   A: U2OS cells. PCRs to amplify the genomic on-target site after *in vitro* cleavage of end-blocked genomic DNA with CRISPR-Cas nucleases targeting RNF2, FANCF, VEGFA and HEKs4. B: HSCs. PCR to amplify the genomic on-target site after *in vitro* cleavage of end-blocked genomic DNA with a VEGFA targeting CRISPR-Cas nuclease. +RNP, cleaved samples; -RNP, untreated samples; ◄, positions of expected PCR amplicons.

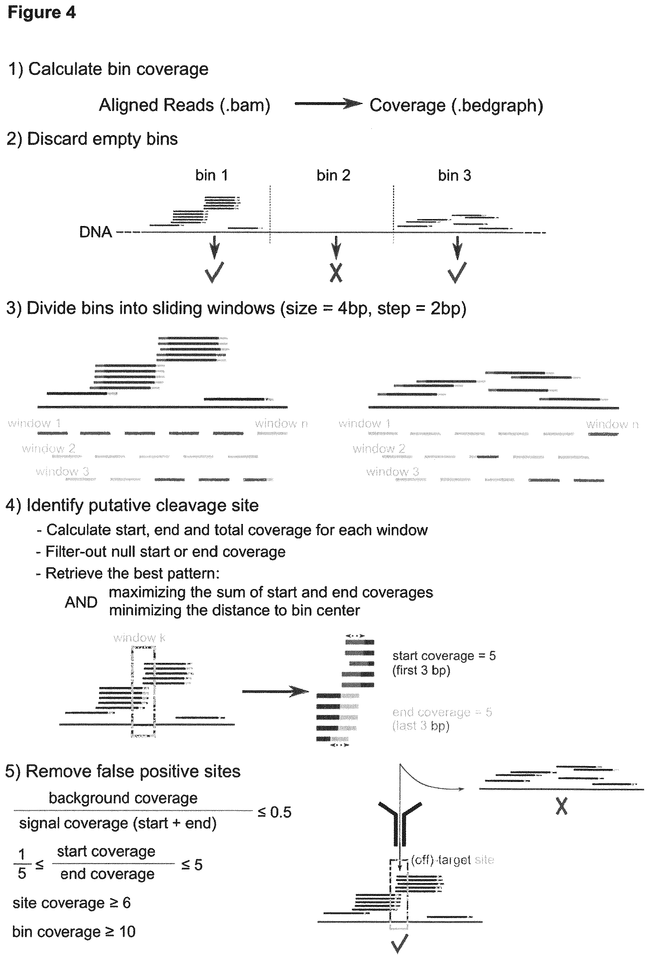

*Figure 4**: Bioinformatic pipeline*
   For identification of target sites, the bioinformatic pipeline followed the steps: (1) calculation of bin coverage, (2) discarding of empty bins, (3) division of bins into sliding windows of 4 nt with 2 nt steps, (4) identification of putative cleavage sites with reads starting or ending at the same site, (5) removing of false positive sites by applying the filters ratio of background coverage to signal coverage ≤ 0.5, ratio of start coverage to end coverage ≥ 1/5 and ≤ 5, site coverage (sum of starting and ending reads) ≥ 6 and bin coverage ≥ 10.

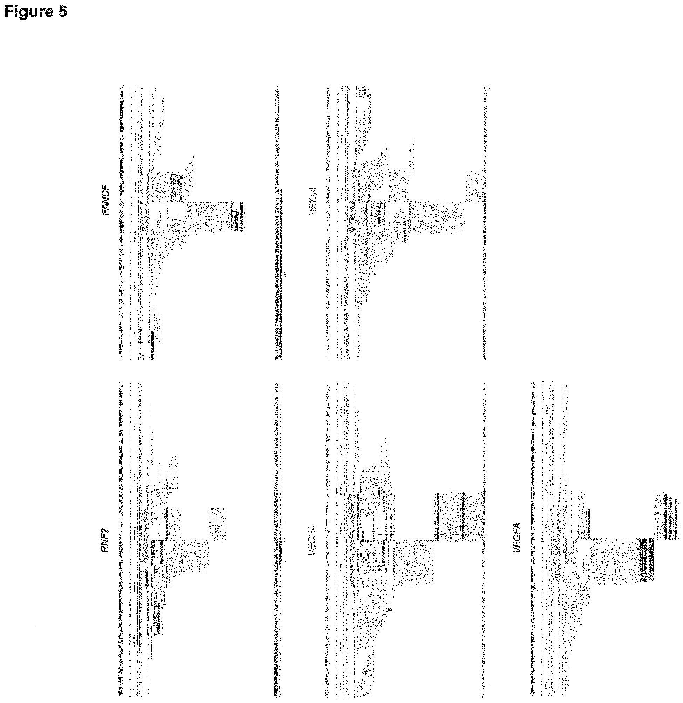

*Figure 5**: Alignment pattern of NGS reads at on-target sites*
   NGS reads were aligned to the human reference genome (hg19) and visualized with Integrative Genomics Viewer (IGV). The chromosomal locations of the respective on-target sites are indicated by a bar. Single reads are shown as arrows, indicating their direction. On the bottom annotated genes (exons as boxes, introns as lines) are indicated. A: Genomic DNA of U2OS cells. B: Genomic DNA of HSCs.

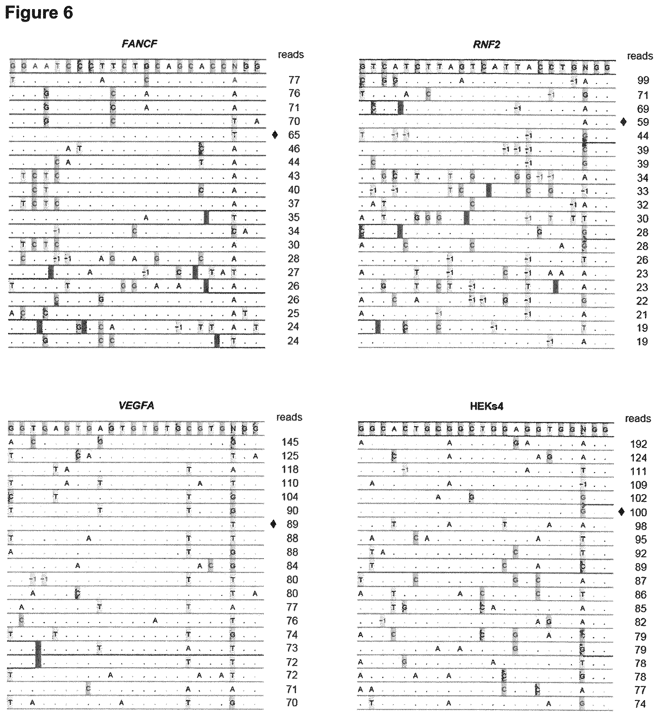

*Figure 6**: Top 20 target sites of tested targets*
   Alignment plots showing the sequences of the top 20 identified target sites, including the on-target site (•) identified for CRISPR-Cas nucleases targeting FANCF, RNF2, VEGFA and HEKs4. The coverage (number of reads) is shown next to the sequence. Top rows show the sequence of the on-target site, including the PAM (NGG). In subjacent rows, the differences to the respective on-target site are highlighted in gray; positive and negative numbers indicate bulges in the DNA or the gRNA, respectively. N, any nucleotide.

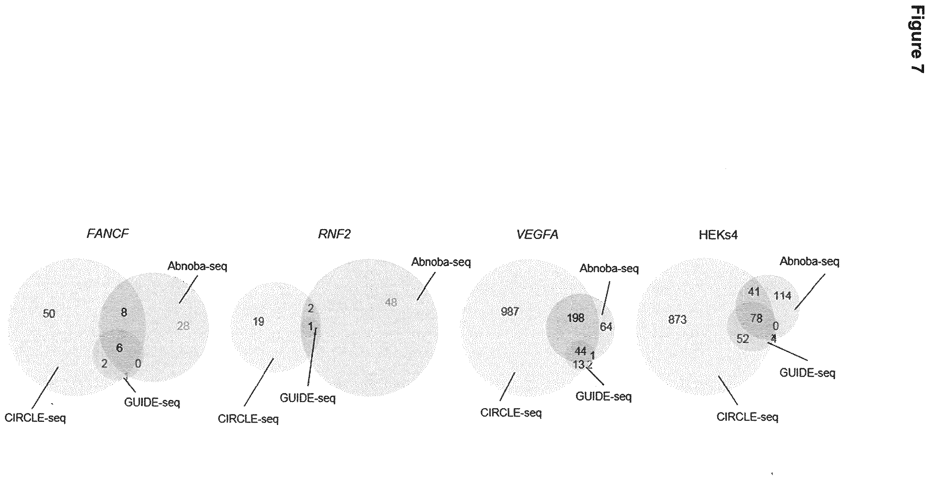

*Figure 7**: Comparison of Abnoba-Seq to GUIDE-Seq and CIRCLE-Seq*
   Venn diagram showing the overlap of identified target sites between Abnoba-Seq, GUIDE-Seq and CIRCLE-Seq.
*Figure 8**: Abnoba-Seq applied to human hematopoietic stem cells*
   A: Top 20 (off-) target sites. Alignment plot showing the sequences of the top 20 identified target sites, including the on-target site (•) identified for a CRISPR-Cas nuclease targeting VEGFA. The coverage (number of reads) is shown next to the sequence. The top row shows the sequence of the on-target site, including PAM. In subjacent rows, the differences to the on-target site are highlighted in gray. Positive or negative numbers indicate bulges in the DNA or in the gRNA, respectively. N, any nucleotide. B: Shared and distinct off-target sites. Venn diagram showing the overlap of identified off-target sites between U2OS cells and HSCs. C, D: Qualitative comparison. Venn diagram showing the overlap of identified top 20 or top 50 target sites between U2OS and HSCs, respectively.

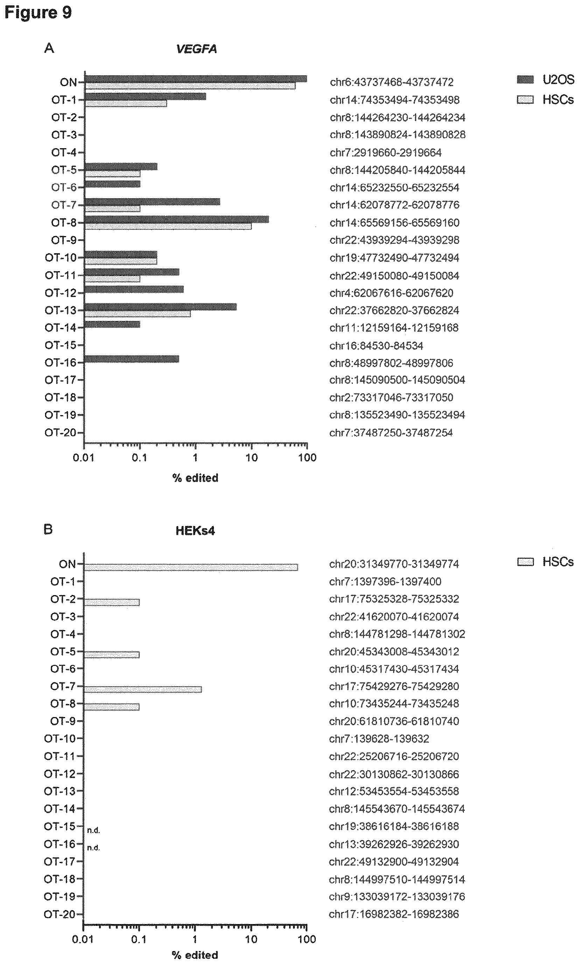

*Figure 9**: Targeted amplicon sequencing of top 20 off-target sites*
   U2OS cells and HSCs were transfected with RNPs targeting VEGFA or HEKs4. Editing was evaluated by targeted amplicon sequencing of the on-target site and the top 20 off-target sites. A: Editing efficiencies with an RNP targeting VEGFA. For each (off-) target site (y-axis) the bar shows the percentage of edited alleles on a logarithmic scale (x-axis) either in U2OS cells (black) or in HSCs (gray). The chromosomal location of each site is indicated. ON, on-target site; OT, off-target sites. B: Editing efficiencies with an RNP targeting HEKs4. For each (off-) target site (y-axis) the bar shows the percentage of edited alleles on a logarithmic scale (x-axis) in HSCs (gray). The chromosomal location of each site is given at the right site. ON, on-target site; OT, off-target sites; n.d., not determined due to sequencing failure.

### DETAILED DESCRIPTION

### Abnoba-Seq

The present invention in a first aspect pertains to method for detecting off-target sites of one or more target-specific programmable nucleases in a genome *in vitro.* This method for detecting off-target sites of one or more target-specific programmable nucleases allows for the detection of all cleavage sites of one or more target-specific programmable nucleases in a genome *in vitro.* Unless otherwise defined, all technical and scientific terms used herein have the same meaning as commonly understood by a skilled person.

The method of the first aspect of the present invention has several advantages compared to known method for determining off-target sites of target-specific programmable nucleases. The method does not use homology to the on-target site/sequence to identify off-target sites, as for example used by known *in vitro* methods, in particular, similarities to the actual target sequence do not play a role for the detection of off-target sites and the method in this regard is completely unbiased. This results in a method with a comparable sensitivity but with a higher selectivity compared to other known *in vitro* methods.

Advantageously, the inventive method can be performed with small amounts of input genomic DNA, which allows for testing of one or more target-specific programmable nucleases on precious cell samples of limited sample size and availability, e.g. by determination of the specificity of target-specific programmable nucleases in primary cells, in biopsy material or a gene edited graft. Moreover, it can be carried out with basic (low-cost) NGS sequencers, not requiring an expensive high-performance sequencer like HiSeq.

The term "target-specific programmable nuclease" is known to the skilled person. Preferably the term "target-specific programmable nuclease" as used in the context of the present invention refers to all forms of nucleases that are capable of recognizing and cleaving a specific site on a desired genome. In particular, it may include, but is not limited to, ZFN, TALEN, meganucleases, Argonaute-family protein-based DNA-guided DNA nucleases and CRISPR/Cas RGN. Terms like "CRISPR/Cas RGN", "ZFN" and "TALEN" may refer to one nuclease or more than one (two, three, etc.) nucleases of that type, for example CRISPR/Cas RGN may refer one CRISPR/Cas RNA-guided nuclease or more than one CRISPR)/Cas RNA-guided nucleases (two, three, etc.), similarly ZFN may refer to one zinc finger nuclease or more than one zinc finger nucleases, etc. In a preferred embodiment a target-specific programmable nuclease is a nuclease that cleaves double stranded DNA (dsDNA) target-specifically, i.e. it targets a specific target site or sequence (on-target site) for cleavage, to preferably introduce a dsDNA break into the DNA.

The term "target site" as used in the context of present invention is known to the skilled person and may preferably refer to on-target site and off-target sites. The term "on-target site" as used in the context of present invention is known to the skilled person. It preferably refers a specific site or sequence, which is targeted by a target-specific programmable nuclease for nucleic acid cleavage, preferably DNA cleavage, and may be selected freely within a nucleotide sequence, preferably a DNA sequence like a genomic DNA sequence, depending on the purpose thereof.

Of course it is understood by the skilled person, that a target-specific programmable nuclease may target nucleic acids, preferably DNA like genomic DNA, for cleavage, in addition to the on-target site, at one or more sites other than the on-target site; these other sites are off-target sites. The term "off-target site" as used in the context of present invention is known to the skilled person. Preferably, the term "off-target site" refers to a site, which is not identical in nucleic acid sequence to the target site or sequence (on-target site) of the target-specific programmable nuclease. That is, it may refer to a site other than the on-target site that is targeted for cleavage by the target-specific programmable nuclease.

Preferably, the off-target site is a site, which is not identical in nucleic acid sequence to the on-target site and which is targeted for cleavage by the target-specific programmable nuclease *in vitro.* In a specific embodiment, the off-target site is a site, which is not identical in nucleic acid sequence to the on-target site and is targeted for cleavage by the target-specific programmable nuclease *in vitro* and *in vivo* (i.e. inside a cell). By targeting for cleavage, it is preferably meant that a target-specific programmable nuclease uses a specific site or sequence to bind directly or indirectly to a nucleic acid, e.g. to a DNA, to then cleave the nucleic acid in, or in close proximity to, this specific site or sequence.

### Step a) - Preparing purified end-blocked dsDNA fragments

The method of the first aspect of the invention comprises the step (a) of providing an input sample comprising genomic DNA, randomly fragmenting the genomic DNA into dsDNA fragments, and ligating a blocking adapter to the ends of the dsDNA fragments and removing dsDNA fragments comprising ends, which are not end-blocked, to prepare purified end-blocked dsDNA fragments.

The genomic DNA in the input sample is not particularly limited as long as it is suitable for use in the method of the present invention. Preferably the genomic DNA is isolated genomic DNA, which means the genomic DNA is extracted from cells, and preferably purified, i.e. separated to some degree, preferably as much as possible, from proteins and other cellular components and/or reagents used during the extraction. The source of the genomic DNA is not particularly limited. The genomic DNA may originate from cells of a specific organism, e.g. from a single or multicellular organism, and for a multicellular organism from cells of a specific tissue, or from a specific cell type. In one embodiment, the genomic DNA originates from eukaryotic cells (e.g. from an animal, a mammal, an insect, a plant, a fungus, an insect, a bird, a fish, an amphibian, a reptile, or a cnidarian).

In a preferred embodiment, the genomic DNA comprised in the input sample provided in step (a) is human genomic DNA, for example from primary cells or biopsy material. Such cells may or may not be cells of a specific tissue, or a specific cell type e.g. of a specific tissue.

The amount genomic DNA in the input sample is not particularly limited. In preferred embodiment of the present invention the input sample of genomic DNA comprises less than 1 µg, preferably less than 900 ng, more preferably less than 800 ng, even more preferably less than 700 ng, even more preferably about 650 ng or less, even more preferably about 600 ng or less, alternatively preferably about 500 ng, alternatively preferably about 400 ng, of genomic DNA.

The term "about", as used herein in combination with a value or range, indicates that the value or range of a given quantity can include quantities ranging within 10% of the stated value or range, or optionally within 5% of the value or range, or in some embodiments within 1% of the value or range. Of course the term "about" also includes the exact value or the exact range of the value or range with which the term is combined.

The way in which the genomic DNA is randomly fragmented into dsDNA fragments, according to the present invention is not particularly limited. Ways to randomly fragment genomic DNA are known in the art. The genomic DNA can for example be randomly fragmented by shearing, i.e. through mechanical breakage of the DNA, or by enzyme-based fragmentation. According to one embodiment of the present invention the genomic DNA is fragmented by sonication (ultrasonication), acoustic shearing, centrifugal shearing, point-sink shearing, needle shearing, or enzyme-based fragmentation, preferably by sonication. Preferably the genomic DNA is fragmented to a defined (median or average) length. The particular length of the genomic DNA into dsDNA fragments is not limited. In a specific embodiment of the first aspect of the present invention the genomic DNA is randomly fragmented to dsDNA fragments with an average or median, preferably median, fragment size of 100 to 2000 bp, preferably 200 to 800 bp, more preferably about 300 to 600 bp, even more preferably 300 to 600 bp, even more preferably about 400 to 500 bp, even more preferably about 400 bp. In a preferred embodiment of the present invention the genomic DNA is randomly fragmented to dsDNA fragments with a median fragment size of about 300 to 600 bp, more preferably 300 to 600 bp, even more preferably about 400 bp, even more preferably 400 bp. The term "bp" refers to one or more base pairs, i.e one or more pairs of complementary nucleotides in a double stranded (ds) nucleic acid, like a dsDNA or a dsRNA. After random fragmentation of the genomic DNA in the input sample to obtain dsDNA fragments, a blocking adapter is ligated to the ends of the dsDNA fragments and then dsDNA fragments comprising ends, which are not end-blocked, are removed to prepare purified end-blocked dsDNA fragments.

The term "blocking adapter" as used herein, refers to a molecule, preferably comprising dsDNA, which, after ligating the blocking adapter to an end of a dsDNA fragment, blocks further ligation as well as cleavage (digestion) by exonucleases from that end. The term "end-blocked" with regard to a dsDNA fragment refers to a dsDNA fragment, to which a blocking adapter has been ligated at both ends.

The blocking adapter is not particularly limited as long as it fulfils the above-described roles. Ways to prepare a blocking adapter, which after ligation blocks further ligation as well as exonuclease cleavage from the end of a DNA fragment to which it is ligated, are known in the art. Preferably, the blocking adapter is a DNA-based adapter, more preferably a dsDNA-based adapter (i.e. dsDNA-based blocking adapter), which means that it comprises dsDNA. Such a dsDNA-based blocking adapter may comprise additional modifications including modified nucleotides and other molecules which are not nucleotides. Such a dsDNA-based blocking adapter preferably has a sequence length, which allows for incorporation of a primer site into the blocking adapter for PCR amplification.

According to a preferred embodiment of the present invention the blocking adapter, preferably a dsDNA-based blocking adapter, comprises one or more modifications, which after ligation of the blocking adapter to a dsDNA fragment, prevent ligation, of the end to which the blocking adapter is ligated to the dsDNA fragment and one or more of modifications, which after ligation of the blocking adapter to a dsDNA fragment, prevent exonuclease cleavage (digestion) of the dsDNA fragment from the end to which the blocking adapter is ligated to the dsDNA fragment.

Modifications in a blocking adapter, which after ligation of the blocking adapter to a dsDNA fragment, prevent ligation, of the end to which the blocking adapter is ligated to the dsDNA fragment are known in the art, and in a dsDNA-based blocking adapter include one or more C3-spacers, one or more Dideoxy-G/Dideoxy-C, or other spacers, preferably one or more C3-spacers, preferably on each strand of the dsDNA-based blocking adapter, preferably at the end of the dsDNA-based blocking adapter, which is not used to ligate the dsDNA-based blocking adapter to the dsDNA fragment (i.e. the end of the blocking adapter which, after ligation, is not directly adjacent to the end of the dsDNA fragment to which the blocking adapter was ligated to).

Modifications in a blocking adapter, which after ligation of the blocking adapter to a dsDNA fragment, prevent exonuclease cleavage of the dsDNA fragment from the end to which the blocking adapter is ligated to the dsDNA fragment are also known in the art, and in a dsDNA-based blocking adapter include one or more phosphorothioate bonds, and one or more C3-spacers, and combinations thereof. In a preferred embodiment the blocking adapter is a dsDNA-based blocking adapter comprising one or more, preferably two or more, phosphorothioate bonds, preferably at least two (e.g. 2, 3, 4, etc.) on each strand of the dsDNA-based blocking adapter, preferably close to the end, e.g. between the last 3-5 nucleotides, of the dsDNA-based blocking adapter that is not used to ligate the dsDNA-based blocking adapter to the dsDNA fragment, (i.e. the end of the blocking adapter which, after ligation, is not directly adjacent to the end of the dsDNA fragment to which the blocking adapter was ligated to); and one or more C3-spacers, preferably at least on each strand of the dsDNA-based blocking adapter, preferably at the end of the dsDNA-based blocking adapter, which is not used to ligate the dsDNA-based blocking adapter to the dsDNA fragment.

The blocking adapter in the form of a dsDNA-based blocking adapter may comprise further modifications including for example one or more, internal deoxyuridines, preferably at least two on each strand, preferably close to the end of the adapter that is not used to ligate the dsDNA-based blocking adapter to the dsDNA fragment. A preferred blocking adapter is a dsDNA-based blocking adapter that comprises a combination of one or more C3-spacers and one or more phosphorothioate bonds, at least one on each strand of the dsDNA-based blocking adapter and preferably at the end of the dsDNA-based blocking adapter, which is not used to ligate the dsDNA-based blocking adapter to the dsDNA fragment, and optionally one or more internal desoxyuridines.

Moreover, the blocking adapter preferably comprises a primer site compatible for use in PCR priming, which means a nucleotide sequence to which a PCR primer can anneal to amplify the dsDNA fragment to which the blocking adapter is ligated. Preferably the sequence of a dsDNA-based blocking adapter and/or the primer site in the blocking adapter is not present in the target genome (i.e. the sequence of the genomic DNA in the input sample and/or the reference genome). In a specific embodiment of the present invention the blocking adapter is a dsDNA-based blocking adapter comprising the forward sequence as defined in SEQ ID NO:01 and the reverse sequence of SEQ ID NO02. In a further specific embodiment of the present invention the blocking adapter is a dsDNA-based blocking adapter consisting of the sequences SEQ ID NO:01 and SEQ ID NO:02.

The blocking adapter is ligated to the ends of the dsDNA fragments by ways known in the art. For example, the blocking adapter can be ligated to the ends of the dsDNA fragment by enzymatic or chemical ligation using a ligation protocol known in the art. Enzymatic ligation can be carried out using a known DNA ligase, which facilitates the joining together of DNA strands by catalysing the formation of a phosphodiester bond, and includes blunt end and sticky end ligation. In a preferred embodiment the blocking adapter is ligated to the ends of the dsDNA fragment by enzymatic ligation.

Preferably, ligating the blocking adapter to the ends of the dsDNA fragments, comprises 1. preparing the dsDNA fragments for blocking adapter ligation; and 2. ligating a blocking adapter to the ends of dsDNA fragments, to obtain end-blocked dsDNA fragments. How to prepare a dsDNA fragment for blocking adapter ligation is known to the skilled person. Preparing the dsDNA fragments for blocking adapter ligation may comprise one or both of end-repairing and dA-tailing (incorporation of a non-templated deoxyadenosine 5'-monophosphate (dAMP) onto the 3' end of blunt-end DNA fragments) the fragmented dsDNA.

In a specific embodiment of the present invention, the dsDNA fragments are prepared for blocking adapter ligation by end repairing, to prepare dsDNA fragments, which are free of overhangs, and contain 5' phosphate and 3' hydroxyl groups, and dA-tailing of thus prepared blunt-end DNA fragments. To the ends of a thus prepared dsDNA fragment a dsDNA-based blocking adapter with a dT-overhang on the 3' end of one end of the adapter can be ligated.

After ligating the blocking adapter to the ends of the dsDNA fragments, dsDNA fragments comprising ends, which are not end-blocked, are removed to prepare purified end-blocked dsDNA fragments. In such dsDNA fragment with ends, which are not end-blocked, one or both ends are not ligated to a blocking adapter. How to remove such dsDNA fragments with unblocked ends is known to the skilled person.

In a preferred embodiment, removing dsDNA fragments comprising ends, which are not end-blocked, comprises contacting the sample comprising the end-blocked dsDNA fragments (after the ligation of blocking adapter to the dsDNA fragments) with one or more exonucleases to remove dsDNA fragments, to obtain purified end-blocked DNA fragments. Suitable exonucleases are known in the art and include for example one or more of exonuclease III, exonuclease V, exonuclease VIII, lambda exonuclease, T5 exonuclease, T7 exonuclease, nuclease BAL-31 and combinations thereof, preferably exonuclease III, lambda exonuclease or combinations thereof. Optionally one or more single-stranded DNA (ssDNA) exonuclease are used, for example selected from exonuclease I, thermostable exonuclease I, exonuclease T, exonuclease VII, RecJf, mung bean nuclease, nuclease P1, and combinations thereof.

In a specific embodiment the one or more exonucleases consists of a mixture exonuclease I, exonuclease III and lambda exonuclease, for example in an enzyme unit ratio of 2 : 20 : 5.

In a preferred embodiment of the method of the first aspect of the present invention step a) comprises the steps of a1) randomly fragmenting the genomic DNA to a defined length to provide dsDNA fragments; a2) protecting the ends of the dsDNA fragments by ligating a blocking adapter to obtain end-blocked dsDNA fragments; and a3) contacting the sample comprising the end-blocked dsDNA fragments with one or more exonucleases to remove dsDNA fragments, which are not end-blocked, to obtain purified end-blocked DNA fragments.

In another specific embodiment of the method of the first aspect of the present invention, in step a) the free ends of remaining not end-blocked DNA fragments are dephosphorylated with a dephosphorylation reagent, preferably after contacting the sample comprising the end-blocked dsDNA fragments with one or more exonucleases to remove dsDNA fragments. In this embodiment preferably in step a) the dephosphorylation reagent consists of one or more phosphatases, the one or more phosphatases preferably comprising, or consisting of, one or more of Antarctic Phosphatase, Calf Intestinal Alkaline Phosphatase, Quick-CIP, Shrimp Alkaline Phosphatase, and combinations thereof.

### Step b) - Preparing a cleaved and amplified DNA library of dsDNA fragments

The method of the first aspect of the invention comprises as a second step, step (b) of preparing a cleaved and amplified DNA library of dsDNA fragments, by b1) cleaving the purified end-blocked dsDNA fragments with one or more target-specific programmable nucleases to obtain cleaved dsDNA fragments; b2) ligating an affinity adapter to cleavage sites of cleaved dsDNA fragments to obtain affinity-adapter-modified dsDNA fragments; b3) enriching for affinity-adapter-modified dsDNA fragments using the affinity adapter to obtain enriched affinity-adapter-modified dsDNA fragments; and b4) amplifying enriched affinity-adapter-modified dsDNA fragments by PCR amplification to obtain a cleaved and amplified DNA library of dsDNA fragments.

According to the present invention, the amount of purified end-blocked dsDNA fragments used in step b1) is not particularly limited. According to a specific embodiment, in step b1) less than 400 ng, preferably less than 300 ng, more preferably less than 200 ng, even more preferably less than 100 ng, for example about 90 ng, about 80 ng, about 70 ng, about 60 ng, less than 60 ng, about 50 ng, or 50 ng, of purified end-blocked dsDNA fragments, alternatively less than 50 ng, e.g. about 40 ng of purified end-blocked dsDNA fragments, are cleaved with the one or more target-specific programmable nucleases to obtain cleaved dsDNA fragments. In preferred embodiment less than 100 ng, preferably for example about 50 ng, of purified end-blocked dsDNA fragments are used in step b1).

According to another specific embodiment of the present invention, in step b1) purified end-blocked dsDNA fragments corresponding to less than 1,500,000 haploid genomes, preferably less than 150,000 haploid genomes, more preferably less than 80,000 haploid genomes, even more preferably less than 60,000 haploid genomes, even more preferably less than 50,000 haploid genomes, for example about 45,000, about 30,000, about 20,000, or about 15,000 haploid genomes, is used.

The purified end-blocked dsDNA fragments may be cleaved with the one or more target-specific programmable nucleases to obtain cleaved dsDNA fragments by contacting a sample comprising the purified end-blocked dsDNA fragments with the one or more target-specific programmable nucleases.

The one or more target-specific programmable nucleases to be used in step b1) are not particularly limited as long as they can be used in the methods of the present invention, which means as long as they are suitable for use as a target-specific programmable nuclease for target-specific cleavage of genomic DNA, e.g. for genome editing, and are suitable for use in the detection of off-target sites using the methods of the present invention, preferably *in vitro* and *in vivo.* Suitable target-specific programmable nucleases include TALEN, ZFN, meganucleases, megaTAL, Fokl-dCas9, Argonaute-family protein-based DNA-guided DNA nucleases, CRISPR/Cas RGN, or a variant thereof, or a combination thereof.

The TALEN that may be used in the methods of the present invention are not particularly limited, as long as they can be used in the methods of the present invention. TALEN refers to one or more fusion proteins comprising a DNA-binding domain derived from Transcription activator-like effector (TALE) proteins and a nucleotide cleavage domain, preferably Fokl cleavage domain. TALE proteins contain multiple 33-35 amino acid repeat domains (TALE repeat arrays) that each recognizes a single base pair (see, e.g., Gaj et al., 2013, Trends Biotechnol., 31(7):397-405). Methods to generate engineered TALE repeat arrays and to fuse them to a nuclease to prepare TALEN are known in the art (see e.g., Reyon et al., 2012, Nature Biotechnology 30, 460-465; Bogdanove and Voytas, 2011, Science 333, 1843-1846; Bogdanove et al., 2010, Curr Opin Plant Biol 13, 394-401; Scholze and Boch, 2011, J. Curr Opin Microbiol 2011; Boch et al.,2009, Science 326, 1509-1512; Cermak et al., 2011, Nucleic Acids Res 39, e82; Tesson et al., 2011, Nat Biotechnol 29, 695-696; Sander et al., 2011, Nat Biotechnol 29, 697-698; and Zhang et al., 2011, Nat Biotechnol 29, 149-153).

The meganucleases that may be used in the methods of the present invention are not particularly limited, as long as they can be used in the methods of the present invention. Meganucleases are sequence-specific endonucleases originating from a variety of organisms such as bacteria, yeast, algae and plant organelles. Endogenous meganucleases have recognition sites of about 12 to 30 bp; customized DNA binding sites with 18 bp and 24 bp-long meganuclease recognition sites have been described, and can be used in the present methods (see e.g. Silva et al., 2011, Current Gene Therapy, 11:11-27; Arnould et al., 2011, Protein Engineering Design & Selection, 24:27-31;). Meganuclease may be selected for example from meganucleases of the LAGLIDADG family, the GIY-YIG family, the His-Cyst box family, and the HNH family. Exemplary meganuclease include I-Scel, I-Ceul, PI-Pspl, PI-Scel, I-SceIV, I-CsmI, I-PanI, I-SceII, I-PpoII, I-SceIII, I-CreI, I-Tevl, I-TevII, and I-Tevlll.

The ZFN that may be used in the methods of the present invention are not particularly limited, as long as they are suitable for used in the methods of the present invention. ZFN are artificially engineered nucleases comprising a zinc-finger DNA binding domain and a DNA cleavage domain. It is possible to artificially engineer the DNA binding characteristics of individual zinc-finger DNA binding domains by randomizing the amino acids at the alpha-helical positions involved in DNA binding and using selection methodologies such as phage display to identify desired variants capable of binding to DNA target sites of interest (Rebar et al., 1994, Science, 263:671; Choo et al., 1994 Proc. Natl. Acad. Sci. USA, 91:11163; Jamieson et al., 1994, Biochemistry 33:5689; Wu et al., 1995 Proc. Natl. Acad. Sci. USA, 92: 344). Such recombinant zinc-finger DNA binding domains can be fused to functional domains, such as nucleases to introduce targeted alterations into genomes of model organisms, plants, and human cells (Carroll, 2008, Gene Ther., 15:1463-68; Cathomen, 2008, Mol. Ther., 16:1200-07; Wu et al., 2007, Cell. Mol. Life Sci., 64:2933-44).

The selection of specific target sites/sequences and the design and construction of ZFN are well known to those skilled in the art, and are described in detail for example in U.S. Patent No 7,888,121; 8,409,861; 6,479,626 ; 6,903,185; and 7,153,949). The ZFN may include any combination of suitable linkers between each zinc finger of the protein. Zinc-finger DNA binding domain may be engineered to be bound to the selected sequence (Beerli et al., 2002, Nat. Biotechnol., 20:135-141; Segal et al., 2003, Biochemistry, 42:2137-48; Mandell et al., 2006, Nucleic Acids Res., 34:W516-523; Carroll et al., 2006, Nat. Protoc. 1:1329-41; Bae et al., 2003, Nat. Biotechnol., 21:275-280; Wright et al., 2006, Nat. Protoc., 1:1637-52; Maeder et al., 2008, Mol. Cell, 31:294-301; Joung et al., 2010, Nat. Methods, 7:91-92). In preferred embodiments, the ZFN are prepared as described in WO 2011/017293 and WO 2004/099366. Additional suitable zinc finger DNA binding domains are described in U.S. Pat. No. 6,511,808; 6,013,453; 6,007,988; and 6,503,717).

The megaTAL and Fokl-dCas9 that may be used in the methods of the present invention are not particularly limited, as long as they are suitable for use in the methods of the present invention. MegaTAL are artificial fusion proteins of a meganuclease with a TAL effector (see e.g. Boissel et al., 2014, Nucl. Acids Res. 42(4):2591-2601; Boissel and Scharenberg, 2015, Methods Mol Biol. 2015; 1239:171-96). Fokl-dCas9 are fusion proteins comprising inactivated Cas9 for sequence targeting and Fokl nuclease (Guilinger et al., 2014, Nat Biotechnol. Jun; 32(6): 577-582; WO 2014/144288; and WO 2014/204578).

The Argonaute-family protein-based DNA-guided DNA nucleases that may be used in the methods of the present invention are not particularly limited, as long as they can be used in the methods of the present invention. Ways to use Argonaute-family protein-based DNA-guided DNA nucleases for genome editing are known in the art (see e.g. WO2017139264A1; Wei et al., 2016, Genes & Diseases, Vol. 3, Iss. 3, p.169-170).

The CRISPR/Cas RNA-guided nucleases (CRISPR/Cas RGN) that may be used in the methods of the present invention are not particularly limited, as long as they can be used in the methods of the present invention. This means that they are at least suitable for use as target-specific programmable nucleases for target-specific cleavage of genomic DNA, e.g. for genome editing, and are suitable for use in the detection of off-target sites using the methods of the present invention, preferably *in vitro* and *in vivo.* CRISPR/Cas RGN refers to the combination of a Cas9 endonuclease and gRNA. The Cas9 endonuclease can bind the gRNA to form a Cas9-gRNA ribonucleoprotein (RNP). Ways to prepare target sequence specific CRISPR/Cas RGN are well known in the art.

The Cas endonuclease of the CRISPR/Cas RGN to be used in the methods of the present invention is not particularly limited, as long as it is suitable for use in a CRISPR/Cas RGN for genome editing and for use in the detection of off-target sites using the methods of the present invention, preferably *in vitro* and *in vivo.* In a specific embodiment of the present invention the Cas endonuclease of the CRISPR/Cas RGN is Cas9 endonuclease or a variant of Cas9 endonuclease or Cpf1 endonuclease or a variant of Cpf1 endonuclease. The Cas endonuclease can be guided via simple nucleotide (nt) complementarity of approximately 20 nucleotides between an engineered gRNA and the complementary strand of a target genomic DNA sequence of interest that lies next to a PAM. The term "nt" as used herein refers to one or more nucleotides (e.g. one, two, three, four, etc.) of a DNA or RNA molecule.

The Cas9 endonuclease, e.g. from S. *pyogenes* (hereafter SpyCas9) can be used (Shen et al., 2013, Cell Res; Jinek et al., 2013, Elife 2, e00471; Hwang et al., 2013, Nat Biotechnol 31, 227-229 (2013); Cong et al., 2013, Science 339, 819-823; Mali et al., 2013, Science 339, 823-826; Cho et al., 2013, Nat Biotechnol 31, 230-232. The engineered CRISPR endonuclease from Prevotella and Francisella 1 Cpf1 (CRISPR-associated endonuclease in Prevotella and Francisella 1) can also be used (see e.g. Zetsche et al., 2015, Cell 163, 759-771; and Makarova et al., 2015, Nat Rev Microbiol 13, 722-736).

In some embodiments, the inventive methods use a wild type or variant of Cas9 endonuclease from the genus *Streptococcus*, the genus *Neisseria*, the genus *Pasteurella*, the genus *Francisella*, the genus *Campylobacter,* e.g. from S. *pyogenes, Staphylococcus aureus, Neisseria meningitidis, Streptococcus thermophilus, or Treponema denticola*; or a wild type or variant of Cpf1 endonuclease from the genus *Lachnospira*, the genus *Butyrivibrio*, *Peregrinibacteria*, the genus *Acidominococcus*, the genus *Porphyromonas*, the genus *Prevotella*, the genus *Francisella*, *Candidatus methanoplasma*, and the genus *Eubacterium*, e.g. from *Candidatus Paceibacter*, *Acidaminococcus sp. BV3L6 or Lachnospiraceae bacterium ND2006;* either as encoded in bacteria or e.g. codon-optimized for expression in mammalian cells and/or modified in its PAM recognition specificity and/or its genome-wide specificity. A number of variants have been described (see e.g. WO 2016/141224, PCT/US2016/049147, Kleinstiver et al., 2016, Nat Biotechnol., 34(8):869-74; Tsai and Joung, 2016, Nat Rev Genet., 17(5):300-12; Kleinstiver et al., 2016, Nature., 529(7587):490-5; Shmakov et al., 2015, Mol Cell., 5; 60(3):385-97; and Tsai et al., 2014, Nat Biotechnol., 32(6):569-76).

The term "gRNA" or "guide RNA" as used herein refers to a target DNA-specific RNA, which may be bound to a Cas endonuclease to form the CRISPR/Cas RGN ribonucleoprotein (RNP), and guides a Cas protein to a target site/sequence. Any type of gRNA can be used in the present methods of the present invention, as long as it is suitable for use in a CRISPR/Cas RGN for genome editing and for use in the detection of off-target sites using the methods of the present invention, preferably *in vitro* and *in vivo.* The gRNA may be e.g. a single guide RNA (sgRNA) or a dual gRNA (dgRNA) comprising a crRNA (CRISPR RNA) /tracrRNA (trans-activating crRNA) pair of RNA molecules. According to a specific embodiment of the present invention the gRNA is a sgRNA, comprising one RNA, or a dgRNA comprising two RNAs, preferably a crRNA and a tracrRNA, as components.

The one or more target-specific programmable nucleases target a specific site or nucleotide sequence (on-target site). The on-target site is not particularly limited and may include one or more specific sites or sequences.

According to one embodiment of the present invention in step b1), the one or more target-specific programmable nucleases are selected from Transcription Activator-Like Effector Nucleases (TALEN), zinc finger nucleases (ZFN), meganucleases, megaTAL, Fokl-dCas9, Clustered Regularly Interspaced Short Palindromic Repeats (CRISPR)/Cas RNA-guided nucleases (CRISPR/Cas RGN) or a variant thereof, or a combination thereof.

In a specific embodiment of the present invention in step b1) the one or more target-specific programmable nucleases comprises, preferably consists of, one or more CRISPR/Cas RGN, each preferably used as a preassembled ribonucleoprotein (RNP), wherein Cas endonuclease is bound to a gRNA, e.g. at a molar ratio of 1:3. The Cas endonuclease may be for example a recombinant protein. According to an exemplary embodiment about 1.6 pmol Cas endonuclease and about 4.8 pmol sgRNA, preferably preassembled into a Cas endonuclease/gRNA RNP is used to cleave about 50 ng purified end-blocked dsDNA fragments; for a larger or smaller amount of purified end-blocked dsDNA fragments the Cas endonuclease and sgRNA used can for example be adapted proportionally.

An in-process control may be used to monitor on-target activity of the one or more target-specific programmable nucleases. According to one embodiment of the present invention PCR amplification using on-target site specific primers for the on-target sites of the one or more target-specific programmable nucleases is used as an in-process control.

The affinity adapter to be used in step b2) of the method of the first aspect of the invention is not particularly limited as long as it allows, after ligation of the affinity adapter to a cleavage site of a purified end-blocked dsDNA fragment, which has been cleaved by the one or more target-specific programmable nucleases (i.e. a cleaved dsDNA fragment), for the specific enrichment of the thus prepared affinity-adapter-modified dsDNA fragment using the affinity adapter. Such affinity adapters are known in the art. Preferably the affinity adapter comprises one or more modifications, which after ligation of the affinity adapter to the cleavage site of a cleaved dsDNA fragment, enable specific enrichment of the affinity-adapter-modified dsDNA fragment.

Preferably the affinity adapter to be used in the inventive methods is a DNA-based adapter, preferably a dsDNA-based adapter (i.e. a dsDNA-based affinity adapter) comprising one or more modifications suitable for affinity purification. Such a dsDNA-based affinity adapter preferably has a sequence length, which allows for incorporation of a primer site for PCR amplification into the affinity adapter.

Modifications suitable for affinity purification are known in the art and include for example biotin and biotin-based modifications, where the biotin or biotin-based modification is attached to the 5' or 3' end of the DNA-based affinity adapter, or internally, or to a specific nucleotide (or nucleotide derivative) of the DNA-based affinity adapter, e.g. to a deoxythymidine. Suitable biotin and biotin-based modifications include for example biotin, biotin dT (biotinylated deoxythymidine base), biotin TEG (15 atom triethylene glycol spacer); desthiobiotin TEG; dual biotin; photocleavable (PC) biotin; biotin azide; nitrilotriacetate (NTA); DNP TEG (2, 4-dinitrophenyl) and combinations thereof. Other suitable modifications include digoxigenin, protein tags, and combinations thereof. Preferably the one or more modifications is selected from one or more, preferably at least two, biotin and biotin-based modifications, and combinations thereof. In a specific embodiment the biotin modifications are added only to one strand of the dsDNA-based affinity adapter.

Moreover, the affinity adapter preferably comprises a primer site compatible for use in PCR priming, which means a nucleotide sequence to which a PCR primer can anneal to amplify the cleaved dsDNA fragment to which the affinity adapter is ligated. Preferably the sequence of a, preferably dsDNA-based, affinity adapter and/or the primer site in the affinity adapter are not present in the target genome (i.e. the sequence of the genomic DNA in the input sample and/or the reference genome).

In a preferred embodiment the affinity adapter is a dsDNA-based affinity adapter, comprising one or more biotin or biotin-based modifications. In a specific preferred embodiment, the affinity adapter is a DNA-based adapter comprising one or more, preferably at least two, internal biotin dT. In a further specific embodiment of the present invention the affinity adapter is a dsDNA-based affinity adapter comprising the sequence as defined in SEQ ID NO:03 and the sequence of SEQ ID NO04. In a further specific embodiment of the present invention the affinity adapter consists of the sequences SEQ ID NO:03 and SEQ ID NO:04.

The affinity adapter is ligated to the cleavage sites of cleaved dsDNA by ways known in the art. The cleavage site of a cleaved dsDNA fragment refers to a cleavage site in a dsDNA fragment, which has been cleaved by the one or more target-specific programmable nucleases. For example, the affinity adapter can be ligated to the cleavage sites of the cleaved dsDNA fragments by enzymatic or chemical ligation using a ligation protocol known in the art. Enzymatic ligation can be carried out using a known DNA ligase. In a preferred embodiment the affinity adapter is ligated to the cleavage sites of cleaved dsDNA by enzymatic ligation.

Preferably, ligating the affinity adapter to the cleavage sites of cleaved dsDNA fragments, comprises 1. preparing the cleaved dsDNA fragments for affinity adapter ligation; and 2. ligating an affinity adapter to cleavage sites of cleaved dsDNA fragments, to obtain affinity-adapter-modified dsDNA fragments. How to prepare a cleaved dsDNA fragment for affinity adapter ligation is known in the art. Preparing the cleaved dsDNA fragments for affinity adapter ligation may comprise one or both of end-repairing and dA-tailing of the cleaved dsDNA.

In a specific embodiment of the present invention, the cleavage sites of cleaved dsDNA fragments are prepared for affinity adapter ligation by end repairing, to prepare cleaved dsDNA fragments, which are free of overhangs, and contain 5' phosphate and 3' hydroxyl groups, and dA-tailing. To the ends of a thus prepared cleaved dsDNA fragment a dsDNA-based affinity adapter with a dT-overhang on the 3' end of one end of the adapter can be ligated.

After ligation the affinity adapter is used to enrich for affinity-adapter-modified dsDNA fragments, to obtain enriched affinity-adapter-modified dsDNA fragments. To "enrich for affinity-adapter-modified dsDNA fragments" in this context refers to the specific, transient or permanent, binding to the affinity-adapter-modified dsDNA fragments via the affinity adapter and separation from dsDNA fragments, which are not affinity-adapter-modified, and optionally other components. Thus the way in which the affinity-adapter-modified dsDNA fragments is enriched for depends on the affinity adapter used (i.e. the type of affinity tag(s) used in the affinity adapter). Protocols to enrich for affinity-adapter-modified dsDNA fragments using a specific affinity adapter are known in the art.

According to a preferred embodiment an affinity adapter binding molecule, which is preferably immobilisable on a solid support, is used to enrich for affinity-adapter-modified dsDNA fragments. An affinity adapter binding molecule refers to a molecule, e.g. protein, antibody, etc., that specifically (reversibly or irreversibly) binds to the affinity adapter. The affinity adapter binding molecule can be immobilized on a on a solid support before or after binding to the affinity adapter. The nature of the affinity adapter binding molecule depends on the affinity adapter used. The affinity adapter binding molecule may be for example a protein. The solid support is not particularly limited and includes for example beads, e.g. magnetic beads. In a preferred embodiment magnetic beads are used as solid support, preferably polymer-coated magnetic beads, like dynabeads.

According to one embodiment, the affinity adapter binding molecule is a protein, preferably selected from streptavidin; avidin; antibodies or functional fragments thereof specific to part of the affinity adapter, e.g. anti-digoxigenin antibodies; digoxigenin binding protein; or a variant thereof; more preferably streptavidin, avidin or a variant thereof. Antibodies or functional fragments thereof specific to part of the affinity adapter preferably bind specifically to one or more modifications suitable for affinity purification on the affinity adapter, e.g. a digoxigenin or a protein tag.

According to a preferred embodiment, the affinity adapter comprises one or more biotins or biotin-based modifications, and the affinity adapter binding molecule is streptavidin, avidin or a variant thereof, preferably streptavidin or a variant thereof. Protocols to enrich for DNA modified with one or more biotin or biotin-based modifications are known in the art. The streptavidin or a variant thereof may be used immobilized on polymer-coated magnetic beads. For example the dynabeads MyOne Streptavidin C1, Dynabeads M-270, Dynabeads M-280, or Dynabeads MyOne Streptavidin T1 can be used.

The enriched affinity-adapter-modified dsDNA fragments are amplified by PCR amplification to obtain a cleaved and amplified DNA library of dsDNA fragments. PCR amplification of dsDNA fragments, including affinity-adapter-modified dsDNA fragments as described herein, is known in the art. In a preferred embodiment, PCR primers specific to parts of the blocking adapter and/or the affinity adapter (i.e. complementary to part of the sequence of the blocking adapter and/or the affinity adapter) are used for PCR amplification.

If the affinity-adapter-modified dsDNA fragments have been enriched using an affinity adapter binding molecule immobilized on a solid support, the affinity-adapter-modified dsDNA fragments bound via the affinity adapter binding molecule to the solid support may serve as template for PCR amplification. Alternatively, the enriched affinity-adapter-modified dsDNA fragments are separated (e.g. released) from the affinity adapter binding molecule and/or the solid support before PCR amplification. In a preferred embodiment, the PCR amplification is carried out using, at least initially, the affinity-adapter-modified dsDNA fragment bound via the affinity adapter binding molecule to the solid support as a template. In this context the term "template" refers to function of an enriched affinity-adapter-modified dsDNA fragment to serve as DNA template to direct synthesis of another DNA or RNA during PCR amplification.

According to a preferred embodiment of the present invention, in step b4) obtaining the cleaved and amplified DNA library of dsDNA fragments comprises a two-step PCR amplification, preferably with 1. a first PCR amplification step for linear PCR amplification using enriched affinity-adapter-modified dsDNA fragments bound via an adapter binding molecule to a solid support as a template and an affinity-adapter-specific primer; and 2. after the first step a second PCR amplification step, after removal of enriched affinity-adapter-modified dsDNA fragments bound via adapter binding molecule to the solid support from the reaction mix and addition of a blocking-adapter-specific primer as a second primer.

### Step c) - Sequencing

The method of the first aspect of the present invention comprises the further step (c) of performing a sequencing of the cleaved and amplified DNA library of dsDNA fragments to obtain sequence reads. How to perform a sequencing of a DNA library of dsDNA fragments is known in the art.

As used herein, "sequencing" includes any method of determining the sequence of a nucleic acid. The method of sequencing to be used in the present methods is not particularly limited and include for example chain terminator (Sanger) sequencing and dye terminator sequencing. In preferred embodiments, next generation sequencing (NGS) is used. Although the different NGS platforms use varying assay chemistries, they generally generate sequence data from a large number of sequencing reactions run simultaneously on a large number of templates. Typically, the sequence data is collected using a scanner, and then assembled and analysed bioinformatically. Thus, the sequencing reactions are performed, read, assembled, and analysed in parallel (see e.g. US 2014/0162897; and MacLean et al., 2009 Nature Rev. Microbiol., 7: 287-296).

Some NGS methods require template amplification and some do not. Amplification-requiring methods include pyrosequencing; the Solexa/Illumina platform; or Supported Oligonucleotide Ligation and Detection (SOLiD) (see e.g. EP 0 946 752; EP 1634 963; EP 0 777 749; EP 1 117 827; and EP 0 777 749; and U.S. Pat. No. 6,969,488; and 6,130,073). Methods that do not require amplification, include for example single-molecule sequencing methods like nanopore sequencing; HeliScope single molecule sequencing; and real-time sequencing by synthesis single molecule real time (SMRT) DNA sequencing methods using zero-mode waveguides (see e.g. EP 2 007 908; EP 1 848 829; EP 2 100 971; and U.S. Pat. No. 7,169,560; 7,501,245; 6,818,395; 7,501,245; and 7,170,050). In a preferred embodiment Illumina sequencing is used.

In a preferred embodiment, in step c) for the sequencing of the cleaved and amplified DNA library of dsDNA fragments, in step c1) the cleaved and amplified DNA library of dsDNA fragments is prepared for sequencing, optionally by ligating sequencing adapters to both ends of the dsDNA fragments; and in step c2) the prepared cleaved and amplified DNA library of dsDNA fragments is then sequenced, preferably by NGS, to obtain the sequence reads. In a specific embodiment, in step c1) preparing the cleaved and amplified DNA library of dsDNA fragments for sequencing comprises preparing cleaved and amplified DNA library of dsDNA fragments for ligation of sequencing adapters to both ends, preferably by one or both of end-repairing and dA-tailing the cleaved and amplified DNA library of dsDNA fragments; followed by ligation of sequencing adapters, preferably to both ends of the dsDNA fragments, and wherein the sequencing adapters are for example Illumina sequencing adapters. In step c2) preferably Droplet Digital PCR (ddPCR), for example with the ddPCR Library Quantification kit for Illumina TruSeq, may be used.

The sequencing in step (c) does not require a high-performance sequencer as for example HiSeq. Instead a basic NGS sequencer like a MiSeq instrument (Illumina), for example using MiSeq Reagent Kit v2, can be used.

### Step d) - Aligning sequence reads to a reference genome

The method of the first aspect of the invention comprises the further step (d) aligning the sequence reads to a reference genome to obtain aligned sequence reads. Alignment in this context means mapping the sequence reads to the corresponding sequences in the reference genome, such that for a specific sequence read the corresponding sequence in the reference genome best (i.e. ranging from an incomplete match to sequence identity) matches the sequence of the sequence read. Ways to align sequence reads to a reference genome are known in the art. For example, any computer program may be used as long as the sequence reads can be aligned as described above, which may be a known program already known in the pertinent art, or a program tailored to the purpose. In a specific embodiment of the present disclosure, the sequence reads are aligned to a reference genome using Bowtie2.

According to a preferred embodiment of the present invention, the genomic DNA is human genomic DNA and the reference genome is a human reference genome, for example selected from hg16 (NCBI Build 34), hg17(NCBI Build 35), hg18 (NCBI Build 36.1), hg19 (GRCh37) and hg38 (GRCh38), preferably selected from hg19 and hg38.

In a specific preferred embodiment, in step d) the sequence reads are aligned to a reference genome to obtain aligned sequence reads, by first in step d1) trimming off blocking adapter sequence, affinity adapter sequence, and optional sequencing adapter sequence from sequence reads to obtain trimmed sequence reads; and second in step d2) mapping the trimmed sequence reads to a reference genome to obtain aligned sequence reads.

### Step e) - Identifying cleavage sites

The method of the first aspect of the invention comprises the further step (e) of identifying cleavage sites, by determining putative cleavage sites and excluding false positive cleavage sites, wherein putative cleavage sites are determined by locating sequence regions in the reference genome where both sequence read starts and sequence read ends of aligned sequence reads coincide, and identifying putative cleavage sites as the sites in these sequence regions, where sequence read starts and sequence read ends meet.

Thus the determination of cleavage sites is not based on sequence homology to the on-target cleavage sites of the one or more target-specific programmable nucleases.

The term "putative cleavage site" may include any cleavage site cleaved by the one or more target-specific programmable nucleases *in vitro* as well as false positive cleavage sites. False positive cleavage sites are sites which during detection of cleavage sites are determined as putative cleavage sites without being a site cleaved by the one or more target-specific programmable nucleases *in vitro* and thus were incorrectly identified, i.e. false positive. The term "putative cleavage site" as used herein refers to a short nt sequence (i.e. a short DNA-sequence in the reference genome), preferably of set length (i.e. not varying from one putative cleavage site to another). In a preferred embodiment a putative cleavage site determined in step e) consists of the nt sequence of a specific window of set sequence width (i.e. a window covering a defined sequence length of the reference genome, which does not vary from one window to another), preferably of a window of about 6 nt or less, more preferably of a window of (about) 4 nt. In other words, in this preferred embodiment the putative cleavage site corresponds to the DNA-sequence in the reference genome covered by the specific window.

Sequence regions in the reference genome where both sequence read starts and sequence read ends of aligned sequence reads coincide are regions in the reference genome of variable or set sequence length, comprising aligned sequence reads, comprising both sequence read starts and sequence read ends, i.e. sequence regions in which, of all the aligned sequence reads in said region that are aligned there to the reference genome, one or more of the aligned sequence reads start and one or more sequence reads end in that sequence region. In a preferred embodiment each sequence region is a bin of defined sequence length, of preferably about 20 to 1000 nt, more preferably of about 50 to 500 nt, even more preferably of at least about 50 nt, even more preferably of at least about 80 nt, most preferably of at least (about) 100 nt. In another preferred embodiment in step e) each sequence region is a bin of defined sequence length, preferably of about 100 nt or a multiple thereof, more preferably of 100 nt or a multiple thereof (i.e. 100 nt, 200 nt, 300 nt, 400 nt, etc.).

A sequence read start refers to one end of an aligned sequence read that is on the opposite side of the sequence read end. For aligned sequence reads aligned to the same nucleotide strand of the reference genome all sequence read starts point in the same direction and all sequence read ends point in the opposite direction of the nucleotide strand of the reference genome sequence. This means, preferably, that if for a first aligned sequence read, which is complementary to a first single stranded sequence of the reference genome the sequence read start is at the 3' end of this first aligned sequence read, for a hypothetical second aligned sequence read, which is of the exact same length and exactly complementary to the first aligned sequence read, the sequence read start would be at the 5' end of second aligned sequence read, such that both aligned sequence reads start at the same spatial location in the reference genome. Thus, according to a specific embodiment of the present invention, any sequence read start of aligned sequence reads aligned to the same continuous sequence of the reference genome point in opposite direction of any sequence read end of aligned sequence reads aligned to that same continuous sequence of the reference genome; i.e. all sequence read starts of aligned sequence reads, aligned to the same continuous sequence of the reference genome, are spatially located in aligned sequence read sequences on one side of the respective sequence centres of individual aligned sequence reads, and all sequence read ends of aligned sequence reads, aligned to the same continuous sequence of the reference genome, are spatially located in the aligned sequence reads sequences on the opposite side of the respective sequence centres of individual aligned sequence reads (compared to the sequence read starts).

In the thus identified sequence regions, putative cleavage sites are identified as the sites in these sequence regions, where sequence read starts and sequence read ends meet. The term "meet" in this context means that the sequence read starts and sequence read ends pointing to each other come into close proximity, i.e. within a few nt, or touch, i.e. neatly align such that no nt separate them on the reference genome sequence to which they reads are aligned to, or overlap by a few nt. Putative cleavage sites in these sequence regions may then be identified for example by finding local maxima of the sum of read starts and read ends of aligned sequence reads in these sequence regions.

In a specific embodiment of the present invention in step e) in each sequence region with a non-zero read start and read end coverage, one or more putative cleavage sites (e.g. exactly one putative cleavage site per sequence region, or alternatively two putative cleavage sites per sequence region, etc.) are determined as the sites, where sequence read starts and sequence read ends of aligned sequence reads coincide and where the sum of the number of sequence read starts and sequence read ends has a local maximum. A non-zero read start and read end coverage means that such a sequence region is a region, where one or more sequence read start and one or more sequence read end, of aligned sequence reads align.

According to a preferred embodiment of the present invention, in step e) putative cleavage sites are determined by firstly dividing (the DNA sequence of) the reference genome comprising aligned sequence reads into bins of a defined length; and secondly determining for each bin with a non-zero read start and read end coverage, one or more putative cleavage sites as sites with a local maximum for the sum of sequence read starts and sequence read ends, and optionally minimizing the distance to the centre of the bin; wherein a sequence read start consists of the first 1 to 5 nt, preferably the first 2 to 4 nt, more preferably the first 3 nt, and a sequence read end consists of the last 1 to 5 nt, preferably the last 2 to 4 nt, more preferably the last 3 nt, of an aligned sequence read. In a specific embodiment of the present invention one putative cleavage sites is determined for each bin.

In a preferred embodiment of the present invention a putative cleavage site determined in step e) consists of the nt of a specific window of set sequence width (i.e. a window covering a defined sequence length of the reference genome, which does not vary from one putative cleavage site to another), preferably of a window of 4 nt. In other words, in this preferred embodiment the putative cleavage site corresponds to the DNA-sequence in the reference genome covered by the specific window. Preferably such a window is a window of defined width, e.g. 4 nt, in a bin, and has a local maximum for the sum of sequence read starts and sequence read ends (aligning in that window). Optionally, the determination of said window includes minimizing the distance to the centre of the bin as an additional step.

A bin with a non-zero read start and read end coverage refers to a bin where one or more sequence read start and one or more sequence read end, of aligned sequence reads align. In the above embodiment, the bin width (i.e. the length of nucleotide sequence covered by the bin) is not particularly limited and may be for example about 20 to 1000 nt, preferably of about 50 to 500 nt, more preferably at least about 50 nt, even more preferably at least about 80 nt. In a preferred embodiment the bin width is at least about 100 nt, preferably at least 100 nt. In another embodiment the bin width is about 100 nt or a multiple thereof, more preferably 100 nt or a multiple thereof (i.e. 100 nt, 200 nt, 300 nt, 400 nt, etc.).

According to a specific embodiment of the present invention in step e) cleavage sites are identified by e1) dividing (the DNA sequence of) the reference genome comprising aligned sequence reads into bins of set sequence length, preferably of about 100 nt length; e2) preferably determining for each bin a read coverage, discarding bins with a read coverage of 0 and preferably combining bins with a read coverage of >0 consecutively, based on the sequence order in the reference genome, into a continuous set of bins; e3) dividing each bin into windows of set (sequence) width, of preferably 4 nt, with a set step size, which is equal or smaller, preferably smaller, than the width of the windows, of preferably 2 nt; e4) determining start, end and background coverage of each window, by grouping,
- sequence read starts falling in the window region into a start coverage group;
- sequence read ends falling in the window region into an end coverage group; and
- sequence reads falling into the window region but not belonging to the start coverage group and the end coverage group into a background coverage group;
and summing up for each window the number of reads in the start coverage, end coverage and background coverage, respectively; e5) determining for each bin comprising at least one sequence read start and at least one sequence read end, one or more putative cleavage sites by maximizing for the sum of start coverage and end coverage and minimizing the distance to the center of the bin; and e6) excluding false positive cleavage sites; wherein a sequence read start consists of the first 1 to 5 nt, preferably the first 2 to 4 nt, more preferably the first 3 nt, and a sequence read end consists of the last 1 to 5 nt, preferably the last 2 to 4 nt, more preferably the last 3 nt, of an aligned sequence read.

Preferably, each of the one or more putative cleavage sites determined in step e5) consists of the nt of a specific window of set sequence width (i.e. a window covering a defined sequence length of the reference genome, which does not vary from one putative cleavage site to another), preferably of a window of 4 nt, set in step e3). Step size as used herein preferably refers to the difference in nt from the first nt of a first window and the first nt of a second window, which is the window positioned just next to the first window along the sequence of the reference genome (i.e. the window positioned closest to the first window when moving from the first window along the sequence of the reference genome in one direction). A set step size refers to a step size that does no vary between different windows positioned closest to each other, e.g. a step size of 2 nt.

In a specific preferred embodiment of the present invention, step e1) comprises dividing (the DNA sequence of) the reference genome comprising aligned sequence reads into bins of set sequence length (preliminary bins), preferably of about 100 nt length; and step e2) comprises determining for each (preliminary) bin of e1) a read coverage, discarding bins with a read coverage of 0 and preferably combining bins with a read coverage of >0 consecutively, based on the sequence order in the reference genome, into a continuous set of bins (final bins); wherein in the continuous set of bins of step e2), preferably, each (final) bin consist of one or a multiple of one of the bins of step e1) (i.e. 1, 2, 3, 4, 5, etc. of the preliminary bins).

Preferably, in this specific preferred embodiment, in the continuous set of bins of step e2), each (final) bin has a bin width, which is defined by the sum of consecutive bins of step e1) between two discarded bins of step e2) closest to each other in the sequence of the reference genome, such that each (final) bin of step e2) starts in the sequence of the reference genome with the bin (i.e. with the start of this bin) of step e1) (preliminary bin), which follows immediately after a first bin discarded in step e2), and ends with the bin (i.e. with the end of this bin) of step e1), which is immediately before the next (i.e. next in the sequence of the reference genome) bin discarded in step e2). This means that in this embodiment, individual (final) bins of step e2) may have a different length (i.e. cover a different length on the sequence of the reference genome), which however is always one or a multiple of one of the bins of step e1) (i.e. 1, 2, 3, 4, 5, etc. bins of step e1)).

In another specific embodiment of the present invention, step e4) further comprises determining start, end and background coverage of each bin, by grouping,
- sequence read starts falling in the bin region into a start coverage group;
- sequence read ends falling in the bin region into an end coverage group; and
- sequence reads falling into the bin region but not belonging to the start coverage group and the end coverage group into a background coverage group;
and summing up for each bin the number of reads in the start coverage, end coverage and background coverage, respectively.

In step e3), the set width of the windows (i.e. the set and constant length of nucleotide sequence of the reference genome covered by each window) and the set step size is not particularly limited. In a preferred embodiment the set width of the windows, which is constant for all windows, is e.g. 8 nt or less, preferably 6 nt or less, most preferably 4 nt; and the set step size is equal or smaller than the width of the windows, preferably smaller than the width of the windows, and is for example 6 nt or less, preferably 4 nt or less (e.g. 4, 3, 2, or 1 nt), most preferably 2 nt. In a specific embodiment one putative cleavage sites is determined for each bin in step e5). In a preferred embodiment of the present invention a putative cleavage site determined in step e) consists of the nt of a specific window (i.e. the sequence covered by a specific window) defined in step e3), preferably a window of 4 nt.

At a specific putative cleavage site, the sequence read starts and sequence read ends, respectively, of the aligned sequence reads of that putative cleavage site, may not align perfectly at a specific position in the reference genome sequence. According to one embodiment, the sequence read starts of aligned sequence reads at a putative cleavage site start within +/- 2 nt or less, preferably +/- 1 nt or less, of a specific first position in the reference genome sequence; and the sequence read ends end of aligned sequence reads at a putative cleavage site end within +/- 2 nt or less, preferably +/- 1 nt or less, of a specific second position in the reference genome sequence; the two specific positions preferably being only a few nt (e.g.1, 2, or 3 nt) apart from each other or directly adjacent to each other in the sequence of the reference genome.

After determination of putative cleavage sites, false positive cleavage sites (herein also referred to as "false positive sites") are excluded from the list of putative cleavage sites to identify cleavage sites. The term "cleavage site" as used herein refers to a short nt sequence (i.e. a short sequence in the reference genome), preferably of set length (i.e. not varying from one putative cleavage site to another). In a preferred embodiment a cleavage site has a sequence length of (about) 4 nt. In specific embodiment a cleavage site identified in step e) consists of the nt of a specific window of set sequence width (i.e. a window covering a defined sequence length of the reference genome, which does not vary from one window to another), preferably of a window of about 6 nt or less, more preferably of a window of (about) 4 nt. In a preferred embodiment of the present invention, false positive cleavage sites are excluded based on the ratio of background coverage to signal coverage at a putative cleavage site; and/or the ratio of start coverage to end coverage at a putative cleavage site; and/or the site coverage; and/or the bin coverage. Preferably, the actual cleavage of a cleavage site of step e) by one of the one or more target-specific programmable nucleases occurs within the sequence of that specific cleavage site, i.e. for a cleavage site, which consist of the nt of a specific window of set sequence width, somewhere in the sequence covered by this specific window.

The start coverage at a putative cleavage site is the sum of sequence read starts aligned in the sequence region of the putative cleavage site, i.e. the sum of aligned sequence reads, which have their sequence read start aligned in the sequence region of the reference genome defined by the putative cleavage site. In a preferred embodiment the start coverage at a putative cleavage site is the sum of sequence read starts aligned in the sequence region of a putative cleavage site, the putative cleavage site being defined by a specific window of set sequence width (i.e. the width does not vary for different windows corresponding to different putative cleavage sites), preferably a window with a sequence width of 4 nt. In other words, in this preferred embodiment, the start coverage at a putative cleavage site is the sum of aligned sequence reads, which have their sequence read start aligned in the sequence region of the reference genome defined by the specific window, of e.g. 4 nt, representing a putative cleavage site (i.e. preferably a window set in step e3) described above).

Similarly, the end coverage at a putative cleavage site is the sum of sequence read ends aligned in the sequence region of the putative cleavage site, i.e. the sum of aligned sequence reads, which have their sequence read end aligned in the sequence region of the reference genome defined by the putative cleavage site. In a preferred embodiment the end coverage at a putative cleavage site is the sum of sequence read ends aligned in the sequence region of a putative cleavage site, the putative cleavage site being defined by a specific window of set sequence width (i.e. the width does not vary for different windows corresponding to different putative cleavage sites), preferably a window with a sequence width of 4 nt. In other words, in this preferred embodiment, the end coverage at a putative cleavage site is the sum of aligned sequence reads, which have their sequence read end aligned in the sequence region of the reference genome defined by the specific window, of e.g. 4 nt, representing a putative cleavage site (i.e. preferably a window set in step e3) described above).

Preferably, the start coverage of a specific window defining a specific putative cleavage site, includes also all sequence read starts, which align only with part of the sequence of the sequence read start in the sequence region of the reference genome defined by the specific window, i.e. it is sufficient that e.g. 1 nt, or 2 nt, etc. of a sequence read start (depending on the length of the sequence read start) align in the sequence region of the specific window for that sequence read start to be included in the start coverage group of a specific window. Similarly, preferably, the end coverage of a specific window defining a specific putative cleavage site, includes also all sequence read ends, which align only with part of the sequence of the sequence read end in the sequence region of the reference genome defined by the specific window, i.e. it is sufficient that e.g. 1 nt, or 2 nt, etc. of a sequence read end (depending on the length of the sequence read end) align in the sequence region of the specific window for that sequence read end to be included in the end coverage group of a specific window.

The background coverage at a putative cleavage site is the sum of sequence reads aligned in the sequence region of the putative cleavage site, but not belonging to the start coverage group and the end coverage group, i.e. the sum of aligned sequence reads aligned in the sequence region of the reference genome defined by the putative cleavage site, without having their sequence read start or sequence read end aligned in the sequence region of the reference genome defined by the putative cleavage site; the putative cleavage site optionally being defined by a specific window of set sequence width (i.e. the width does not vary for different windows corresponding to different putative cleavage sites), preferably a window with a sequence width of 4 nt (i.e. preferably a window set in step e3) described above).

The term "site coverage" as used herein refers to the sum of start coverage and end coverage at a putative cleavage site, i.e. the sum of sequence read starts and sequence read ends of aligned sequence reads, meeting/coinciding at a putative cleavage site. In a preferred embodiment the term "site coverage" refers to the sum of start coverage and end coverage at a putative cleavage site, the a putative cleavage site being defined by a specific window of set sequence width (i.e. the width does not vary for different windows corresponding to different putative cleavage sites), preferably a window with a sequence width of 4 nt; in other words the term "site coverage" refers to the sum of sequence read starts and sequence read ends of aligned sequence reads, aligned to the reference genome in the sequence region of a specific window of set sequence width, of e.g. 4 nt, representing a specific putative cleavage site (i.e. preferably a window set in step e3) described above). The term "bin coverage" as used herein refers to the sum of all aligned sequence reads in a bin (i.e. the sum of all aligned sequence reads that align in the sequence region of the reference genome of a specific bin).

In another preferred embodiment of the present invention, false positive cleavage sites are excluded by selecting only putative cleavage sites with 1.) a ratio of background coverage to signal coverage, which does not exceed a certain threshold, preferably about 0.5; and/or 2.) a ratio of start coverage to end coverage at the putative cleavage site, falling within a certain numerical range, preferably of between about 1/5 and 5/1; and/or 3.) a minimum site coverage, of preferably about 6 or more; and/or 4.) a minimum bin coverage, of preferably 10 or more.

In a specific embodiment of the present invention false positive cleavage sites are excluded by selecting only putative cleavage sites with 1. a ratio of background coverage to signal coverage ratio of 0.5 or less at the putative cleavage site; and 2. a ratio of start coverage to end coverage of between 1/5 and 5/1 at the putative cleavage site; and 3. a site coverage of 6 or more; and 4. a bin coverage of 10 or more.

According to yet another specific alternative embodiment of the present invention, false positive cleavage sites are excluded or further excluded by using a machine learning model, preferably trained on false positive sites excluded by the procedure described in the embodiments above, preferably trained using ratio of background coverage to signal coverage; ratio of start coverage to end coverage; bin width; relative distance between false positive sites; and/or ratio of false positive sites to bins as features. How to use a machine learning model, including selection of a suitable model, selection of features and training is known in the art. For example, a random forest model may be built, e.g. using a 3-fold 10 repeats cross validation and by obtaining an area under the curve of 1.

In one embodiment of the present invention each cleavage site of step e) constitutes one target site (i.e. one on-target site or one off-target site) of the one or more target-specific programmable nucleases, such that preferably each target site (i.e. on-target site or off-target site) consists of one specific cleavage site, which is different for each target site (i.e. one of the individual cleavage sites identified in step e)). In a specific embodiment, where a specific cleavage site of step e) consists of the nt of a specific window of set sequence width, each target site (i.e. on-target site or off-target site) consists of the sequence of a specific window of set sequence width defining one specific cleavage site, such that each specific window, defining one specific cleavage site, identified in step e), gives rise to one target site.

According to another embodiment of the present invention the cleavage sites of step e) are comprised in the target sites (i.e. on-target site or off-target site) of the one or more one or more target-specific programmable nucleases, such that each target site (i.e. on-target site or off-target site) comprises one cleavage site. It is understood by the skilled person that, in this embodiment, a cleavage site determined in step e), which may consist of the nt of a specific window of set sequence width of step e), does not necessarily constitute the whole target site sequence (i.e. on-target site or off-target site sequence), since for example an on-target site may further comprise e.g. a target sequence (i.e. the sequence a specific target-specific programmable nucleases is designed to recognize) and/or a nuclease binding sequence (for CRISPR/Cas RGN e.g. a PAM sequence); similarly an off-target site may, in this embodiment, for example further comprise e.g. a sequence which is, to a certain degree (even a very small degree, e.g. 1 nt, or 2 nt, or 3 nt, etc. sequence homology), homologous to a target sequence and/or a nuclease binding sequence.

A CRISPR/Cas RGN target site, in this other embodiment, may comprise in addition to the cleavage site a target sequence and a PAM sequence. For example, if one or more target-specific programmable nucleases used in step b) consist of one or more CRISPR/Cas RGN, a target site, in this other embodiment, may comprise or consist of a target sequence, e.g. of about 18-23 nt, preferably about 20 nt, which is identical, or homologous to at least a very low degree (i.e., e.g. 1 nt, or 2 nt, or 3 nt, or 4 nt, or 5 nt, or 6 nt, or 7 nt, or 8 nt etc. sequence homology), to the target sequence of one of the one or more target-specific programmable nucleases used in step b); and a PAM sequence, e.g. of 2-6 nt, preferably of 3 nt, directly adjacent to the target sequence. Thus, in this other embodiment, in a specific preferred embodiment where the one or more target-specific programmable nucleases consist of one or more CRISPR/Cas RGN, the target sites, preferably the off-target sites, comprise, or consist of, a sequence of e.g. about 20-29 nt length, preferably about 23 nt. Such sequences of e.g. about 23 nt can be, for example, identified by identifying cleavage sites of step e) that have in close proximity or overlap with their sequence with 1) a PAM sequence of 2-6 nt preferably 3 nt, and 2) a target sequence, which is at least to a certain, preferably very low, degree homologous (e.g. at least 1 nt, or at least 2nt, or at least 3nt, or at least 4nt, or at least 5 nt, or at least 6 nt, or at least 7 nt, or at least 8 nt, or at least 9 nt, or at least 10 nt, or at least 11 nt, or at least 12 nt, or at least 13 nt, etc. sequence homology) to a target sequence of one of the of one or more CRISPR/Cas RGN used in step b). The cleavage site preferably is located in such a target site (i.e. on-target site or off-target site) of e.g. 23 nt length, close to the end of the target sequence directly adjacent to the PAM sequence, e.g. within a sequence range consisting of the first about 1-7 nt of the end of the target sequence directly adjacent to the PAM sequence and the about first 1-2 nt of the end of PAM sequence directly adjacent to the target sequence.

According to a specific embodiment of the method of the first aspect of the present invention, the off-target cleavage sites of the one or more target-specific programmable nucleases are identified as cleavage sites of step (e) that are not on-target cleavage sites of the one or more target-specific programmable nucleases. According to an alternative specific embodiment of the method of the first aspect of the present invention, the off-target cleavage sites of the one or more target-specific programmable nucleases are identified as target sites comprising a cleavage sites of step (e) that are not on-target sites of the one or more target-specific programmable nucleases. In practice this can be done by identifying (e.g. by labelling) in the list of cleavage sites identified in step e) the on-target sites of the one or more target-specific programmable nucleases, which are known as a result of designing the one or more target-specific programmable nucleases. This can be done by means known in the art.

### Dual-Abnoba-Seg

In a second aspect the present invention pertains to a method for detecting *in vivo* off-target sites by using the method for detecting off-target sites of one or more target-specific programmable nucleases in a genome *in vitro* of the first aspect (Dual-Abnoba-Seq). In particular, the second aspect of the present invention relates to a method for detecting off-target sites in a genome *in vivo* comprising the steps of i) providing a first sample, comprising genomic DNA of cells treated with one or more target-specific programmable nucleases, and a second sample, comprising genomic DNA of cells of related genetic background, preferably cells of the same genetic background, not treated with the one or more target-specific programmable nucleases; ii) for both samples independently detecting off-target sites *in vitro* by using the method of the first aspect of the present invention as defined in any one of the embodiments described above and using the one or more target-specific programmable nucleases of step i); and iii) comparing off-target sites between the two samples to identify off-target sites only identified in the sample comprising genomic DNA of cells not treated with the one or more target-specific programmable nucleases, but not in the sample comprising genomic DNA of cells treated with the one or more target-specific programmable nucleases as *in vivo* off-target sites of the one or more target-specific programmable nucleases.

The method according to the second aspect of the invention allows for individualized determination of *in vivo* specificity of one or more target-specific programmable nucleases, in other words the ability to determine actual off-target activity of the one or more target-specific programmable nucleases in specific cells. This means that the method according to the second aspect of the present invention can be used to demonstrate target-specific programmable nucleases activity *in vivo* e.g. in a particular cell type.

The present inventor found that by isolating genomic DNA from gene-edited cells and untreated cells and for both these samples of genomic DNA carrying out the method for detecting off-target sites of one or more target-specific programmable nucleases in a genome *in vitro* according to the first aspect of the invention with the target-specific programmable nucleases used for gene-editing in the gene-edited cells, allows the determination of the actual off-target activity in cells.

By comparison of the off-target sites identified in the gene-edited sample of genomic DNA with the off-target sites in the sample of genomic DNA from the untreated cells, the actual *in vivo* activity of one or more target-specific programmable nucleases for example in cells of a particular cell type or tissue can be determined as those sites no longer identified in the sample of genomic DNA derived from the gene-edited cells, using minimal amounts of genomic DNA, for example of less than 1 µg per sample, thereby allowing for the testing of *in vivo* off-target effects in precious cell samples of very small sample size and limited availability.

Ways to treat cells with one or more target-specific programmable nucleases are known in the art (see e.g. Yin et al., 2017, Delivery technologies for genome editing. Nat Rev Drug Discov 16, 387-399). For that the one or more target-specific programmable nucleases are introduced into the cells by means known in the art and at concentrations and in a form suitable for genome editing *in vivo.* For example, if the one or more target-specific programmable nucleases comprises one or more CRISPR/Cas RGN, for example the gRNA, the Cas endonuclease, or both, can be introduced as a purified protein and/or nucleic acid into the cells, or can be expressed transiently or stably in the cell, using methods known in the art. The one or more target-specific programmable nucleases may be one of those specified above in the first embodiment of the present invention.

In particular this second aspect of the invention pertains to a method for detecting off-target sites in a genome *in vivo* comprising the steps of i) providing a first sample, comprising genomic DNA of cells treated with one or more target-specific programmable nucleases, and a second sample, comprising genomic DNA of cells of related genetic background, preferably cells of the same genetic background, not treated with the one or more target-specific programmable nucleases. Preferably, a related genetic background means that such cells have the same genetic background, which means that they have as little differences as possible, ideally an identical genetic code, i.e. the genomic DNA of these cells is the same except for minor variations that may occur from one cell to another for example due to random mutations. Preferably, the cells related genetic background are preferably cells originate from the same anatomical region in a multicellular organism, e.g. a human body, e.g. from the same tissue and/or the same cell type.

In a preferred embodiment of the method of the second aspect of the present invention, cells of related genetic background are cells of the same genetic background, preferably with the same genetic code, and preferably with the same anatomical origin, preferably cells originating from the same tissue and/or cell type. In another embodiment the cells of related genetic background are cells of the same cell lineage. Cells of the same cell lineage as used herein, very generally refer to cells derived from a common ancestor cell that are the result of mitotic cell division and optionally cell differentiation. Preferably, cells of the same cell lineage are cells originating from the same tissue, and preferably cells of the same cell type.

Preferably cells of related genetic background are cells originally derived from the same individual organism. The type of organism from which the cells of the first and second sample originate is not particularly limited as long as the cells are suitable for use in the method of the second aspect of the invention. In one embodiment, the cells are eukaryotic cells (e.g. from an animal, a mammal, an insect, a plant, a fungus, an insect, a bird, a fish, an amphibian, a reptile, or a cnidarian). In a specific embodiment the cells are human cells. For example the cells may be primary cells.

For both samples independently off-target sites *in vitro* are detected by using the method of the first aspect of the present invention and using the one or more target-specific programmable nucleases of step i). Finally, off-target sites between the two samples are compared to identify off-target sites only identified in the sample comprising genomic DNA of cells not treated with the one or more target-specific programmable nucleases but not in the sample comprising genomic DNA of cells treated with the one or more target-specific programmable nucleases as *in vivo* off-target sites of the one or more target-specific programmable nucleases. Ways to compare off-target sites between the two samples are known in the art. Preferably this is done using a computer program, existing or specifically written for this purpose. For example, after normalization of the bin coverage by the bin width, the cleavage sites can be sorted by their location in the genome and results of edited cells written next to results of non-treated cells.

In a further aspect the present invention pertains a kit for carrying out the method according to the first aspect of the present invention as defined in any of the embodiment described above, and a kit for carrying out the method according to the second aspect of the present invention as defined in any of the embodiment described above. Ways to prepare such kits are known in the art-

The following examples further explain the invention.

### EXAMPLES

### Materials and methods applied in the examples

### Ribonucleoprotein complex formation

For *in vitro* experiments, gRNAs were transcribed *in vitro* from PCR amplicons using the HiScribe T7 High Yield RNA Syntesis kit (NEB) followed by cleaning up with the MEGAclear kit (Thermo Fisher Scientific). For cell-based experiments, chemically modified gRNAs were purchased (Synthego). Cas9 protein was produced in-house according a previously published protocol (Jinek et al, 2012, Science 337(6096): 816-821). To allow for RNP complex formation, Cas9 and gRNA were incubated at room temperature for 10 min at a molar ratio of 1:3 Cas9 protein to gRNA.

### Cell culture and transfection

U2OS cells were cultivated in DMEM with low glucose and pyruvate (Gibco) supplemented with 10% FBS and 1% penicillin/streptomycin (Sigma-Aldrich). CD34+ cells were purified from human cord blood obtained from the Department of Obstetrics and Gynecology with informed consent, using Ficoll-based density gradient centrifugation and subsequent positive selection using the human CD34 MicroBead Kit UltraPure (Miltenyi Biotec). CD34+ cells were cultured in CellGro SCGM (CellGenix^{®}) supplemented with 20 ng/µl TPO, 60 ng/µl SCF and 60 ng/µl Flt-3L (CellGenix^{®}). Before electroporation, 12 ng/µl of IL-3 was added to the CD34+ cell culture medium. Cells were transfected with RNPs using a Nucleofector 4D (Lonza Group AG) using either SE kit and program DN-100 for U2OS cells or P3 kit and program CA-136 for CD34+ cells according to instructions of the manufacturer.

### Isolation and fragmentation of genomic DNA

Genomic DNA of cells was isolated using the QIAamp DNA Blood Mini Kit (Qiagen). Shearing of genomic DNA to a median length of 400 bp was performed with a M220 focused-ultrasonicator (Covaris) according to the manufacturer's instructions.

### Abnoba-Seq workflow

All primers used are indicated in Table 1. Throughout the work flow, the NEBNext Ultra II kit (NEB) and AMPure XP beads (Beckman Coulter) were used. Creation of batch of end-blocked DNA fragments: Sheared genomic DNA (600 ng per reaction) was end-prepped and blocking-adapter (112.5 pmol) was ligated using the NEBNext Ultra II kit (NEB) for 30 min at 20°C. Not end-blocked fragments were removed by exonuclease treatment with Exonuclease III (200 U, NEB), Exonuclease I (20 U, NEB) and Lambda Exonuclease (50 U, NEB).

*In vitro* cleavage: 50 ng end-blocked genomic DNA fragments were cleaved *in vitro* with 1.6 pmol Cas9 protein (lab-made) and 4.8 pmol gRNA in a reaction volume of 25 µl with 1x NEB Buffer 3 and 1x BSA for 1 h at 37°C. The reaction was stopped by adding 4 µg of RNase and STOP solution containing 50% EDTA 0.5 M pH 8.0, 30% glycerol and 1% SDS, incubated for 15 min at 37°C each. Quality control PCR was performed using Q5 Polymerase (NEB) and the on-target sites were amplified.

Biotinylation of cleaved DNA fragments: Cleaved DNA fragments were end-prepped and biotin-adapter (75 pmol) was ligated using the NEBNext Ultra II kit for 30 min at 20°C. Biotinylated fragments were enriched via Dynabeads MyOne Streptavidin C1 (Thermo Fisher Scientific) according to the manufacturer's protocol. Afterwards, streptavidin-bound biotinylated DNA fragments were applied as template to linear PCR amplification by use of only a biotin-adapter-specific primer. After 10 cycles, the PCR reaction was paused at 4°C and the PCR reaction with linear PCR products transferred to a new tube, blocking-adapter-specific primer was added and the PCR continued for further 10 cycles. Amplicons were applied to DNA library preparation to prepare them for next generation sequencing.

Next generation sequencing: DNA libraries were quantified using ddPCR with the ddPCR Library Quantification kit for Illumina TruSeq (Bio-Rad) and sequencing was performed on an MiSeq instrument (Illumina) using the MiSeq Reagent Kit v2 (300 cycles).

Off-target analysis: First steps of sequencing data analysis and some of the later steps were done using Galaxy at usegalaxy.eu (Afgan et al., 2018, Nucleic Acids Res 46(W1): W537-W544). First, adapter sequences from Illumina-adapter, blocking-adapter and biotin-adapter were trimmed off the reads by use of Trim Galore with default settings. Trimmed reads were mapped to the human reference genome (hg19) with Bowtie2 with default settings. Afterwards, aligned sequence reads were further analyzed by first quantifying the total read coverage within a 100 nt sliding window over the human genome using bedtools (Quinlan and Hall, 2010, Bioinformatics 26(6): 841-842). The non-empty consecutive windows with a coverage of >0 were merged into bins, whereas empty windows were discarded. Next, bins were divided into overlapping windows of 4 nt with a step of 2 nt. The first and last nucleotides (nt) of each read were considered to calculate start and end coverages, respectively. Reads which neither belonged to the group of start coverage nor to the group of end coverage were considered for the background coverage. For every single bin with a non-null start and end coverage, a putative cleavage site was retrieved by maximizing the start and end coverage and minimizing the distance to the center of the bin. False positive sites were filtered out by applying the criteria background to signal ratio ≤ 0.5, start to end ratio ≥ 1/5 and ≤ 5, site coverage (start and end coverage) ≥ 6 and bin coverage ≥ 10. In order to further eliminate remaining false positive sites, a machine learning model was trained on RNF2 annotated sites based on the features start to end ratio, background to signal ratio, bin width, relative distance and site to bin ratio. RNF2 sites identified by the used pipeline were classified as true or false positive based on literature and prior knowledge. A random forest model was built on two-thirds of these sites using a 3-fold 10 repeats cross validation and obtained an area under the curve of 1, on both training and testing sets. The trained model was then used to classify the other target sites and the predicted false positive sites were filtered out.

Visualization: For visualization of identified target sites, the Integrative Genomics Viewer (IGV) was used (Robinson et al., 2011, Nat Biotechnol 29(1): 24-26; Thorvaldsdottir et al., 2013, Brief Bioinform 14(2): 178-192).

### Targeted amplicon sequencing

U2OS cells and CD34+ cells isolated from cord blood were nucleofected with RNPs targeting VEGFA and HEKs4, and genomic DNA of treated and untreated cells harvested on day 7 after transfection. The on-target site, as well as the top 20 off-target sites of the CRISPR-Cas9 nucleases were amplified by PCR with amplicon sizes of around 300 bp using Q5 Polymerase (NEB). PCR amplicons were purified using the QIAquick PCR Purification Kit (Qiagen) and concentration was measured using the Qubit Fluorometer (Thermo Fisher Scientific). 20 ng each of the 21 amplicons were mixed in groups (untreated/gene edited, U2OS/HSCs) and applied to DNA library preparation using the NEBNext Ultra II kit (NEB). DNA libraries were quantified using ddPCR with the ddPCR Library Quantification kit for Illumina TruSeq (Bio-Rad) and sequencing was performed on an MiSeq (Illumina) using the MiSeq Reagent Kit v2 (500 cycles). After NGS, reads were analysed using CRISPResso (Pinello et al., 2016, Nat Biotechnol 34(7): 695-697) with the following settings: filter reads based on the phred33 quality score with the minimum average quality score set to 30 (q 30); trim Illumina adapter sequence from the reads; quantify indels within a window of 40 nt around the predicted cleavage site (w 40); ignore substitutions events for the quantification in order to not consider SNPs. Editing efficiency was calculated by use of the formula: % edited = % indel reads in gene-edited cells - % indel reads in untreated cells

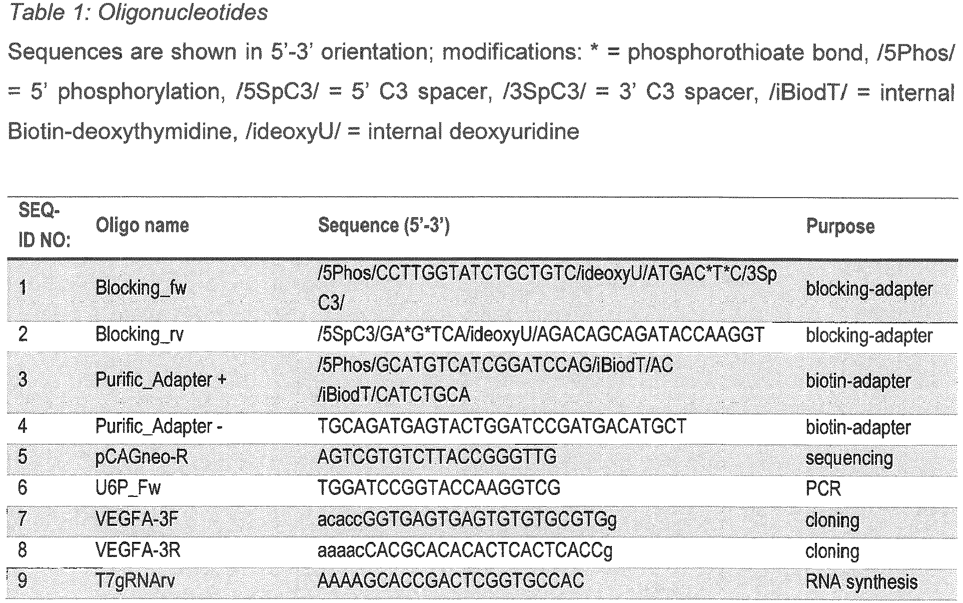

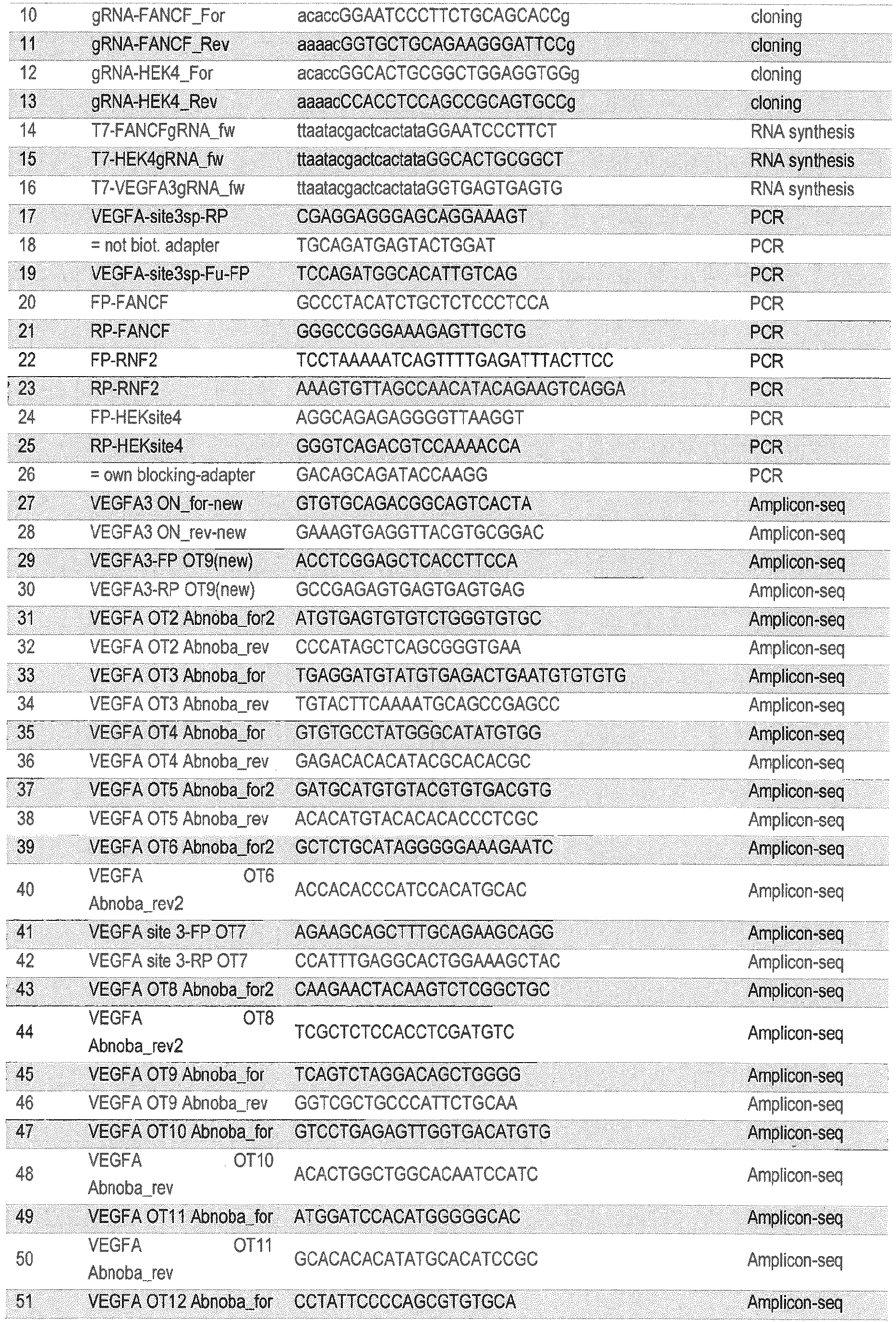

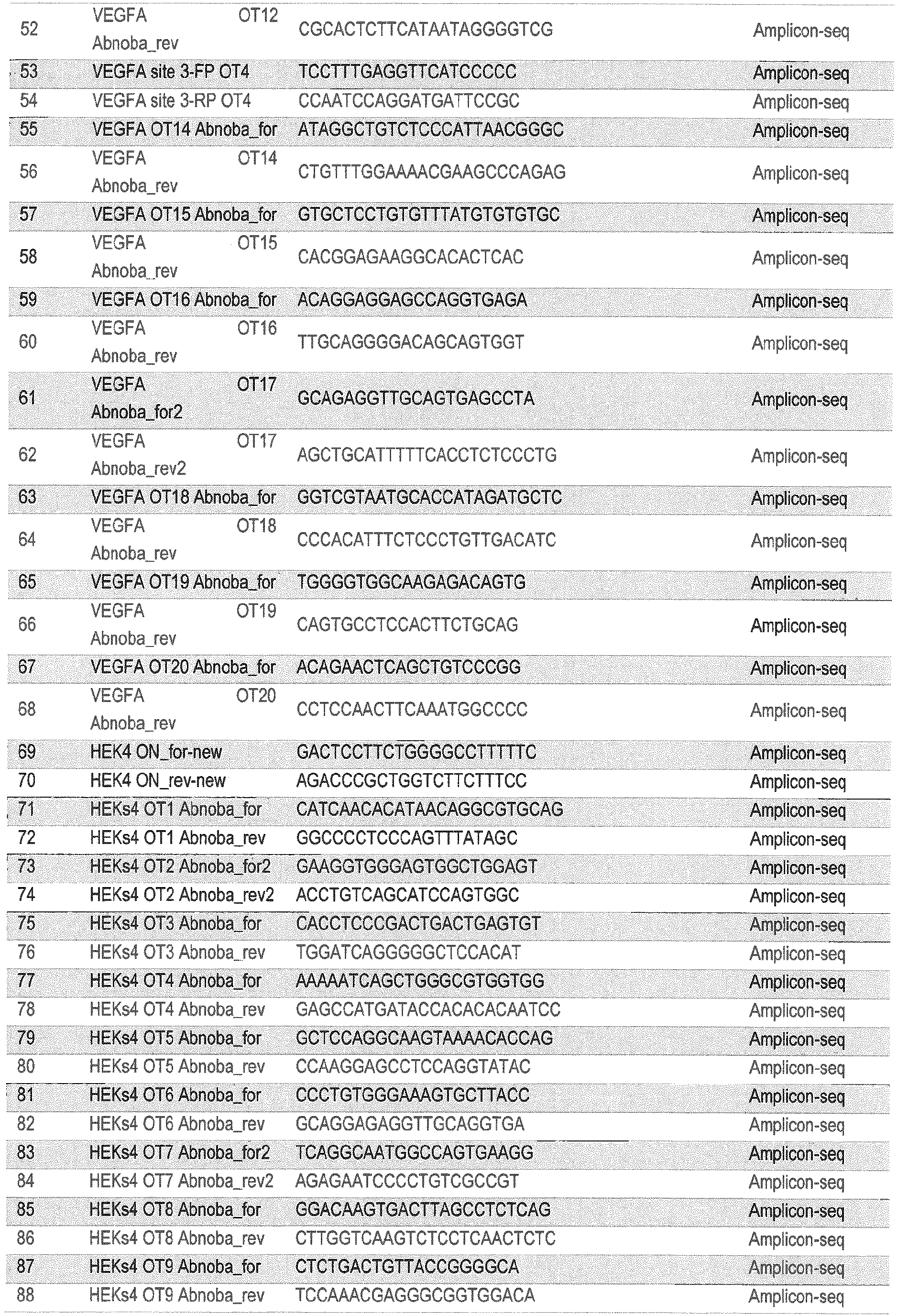

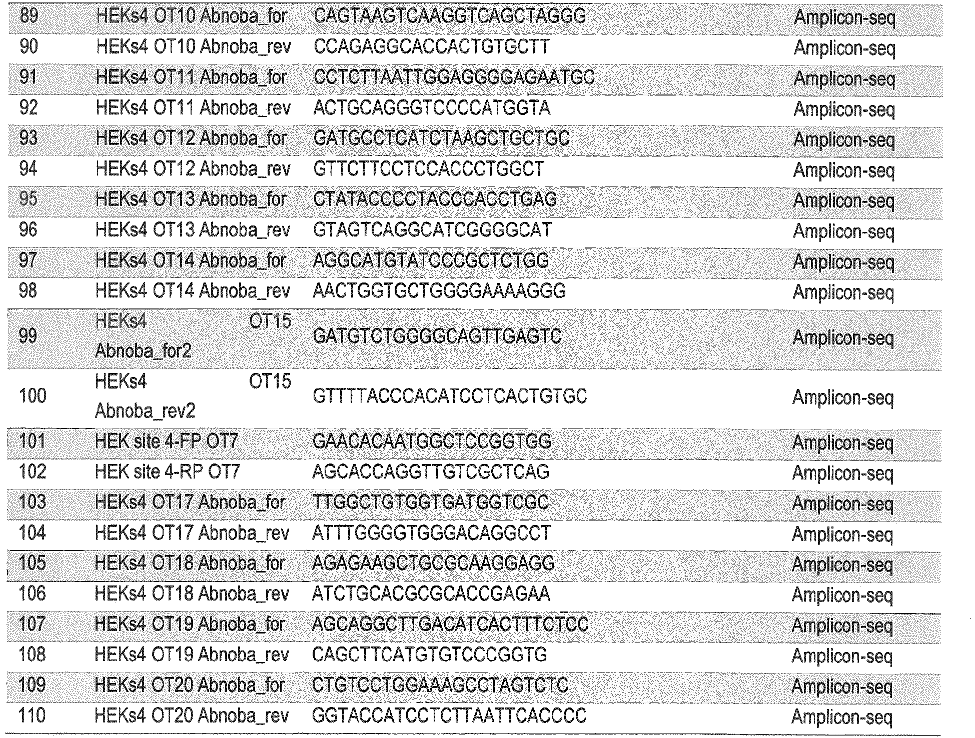

### Results

### Example 1: Abnoba-Seq workflow

The present inventors hypothesized that cleavage of fragmented and end-blocked genomic DNA with CRISPR-Cas *in vitro* will enable subsequent tagging of the cut sites with biotinylated adapters, which in turn will allow for easy enrichment of cleaved DNA fragments and final identification of target sites after high-throughput sequencing (Figure 1). To generate a fragmented and end-blocked genomic DNA library, genomic DNA was randomly sheared to a median size of -400 bp, and a double-stranded (ds) DNA adapter with C3-spacers on both strands was ligated to the end-repaired and dA-tailed DNA fragments. DNA fragments with blocking-adapter ligated to both ends, here referred to as end-blocked fragments, accounted for more than 99% after treatment of the fragments with a combination of Exonuclease III, Exonuclease I and Lambda Exonuclease (Figure 2). 50 ng of end-blocked DNA fragments, corresponding to -15,000 haploid genomes, were then cleaved with 1.6 pmol of RNPs (SpyCas9 protein complexed to gRNA at a molar ratio of 1:3). The cleavage reaction created free DNA ends at all target sites, to which a dsDNA-based affinity adapter with two internal biotinylated dT bases was ligated after end-repairing and dA-tailing. As an in-process control, CRISPR-Cas9 on-target activity was monitored by PCR amplification using on-target site specific primers. If the on-target site was cleaved effectively, PCR should return only weak bands (Figure 3). If the samples passed the in-process control, beads loaded with streptavidin, which has a high affinity for biotin, were used to enrich for CRISPR-Cas cleaved DNA fragments. These adapters also added target sequences for subsequent PCR steps that were used first to release biotinylated DNA fragments from the streptavidin beads by linear amplification and second to create dsDNA again. Finally, a sequencing library with Illumina adapters was generated and subjected to paired-end next-generation sequencing (NGS). Importantly, the Abnoba-Seq pipeline is not based on a sequence homology-driven algorithm. As a consequence, it stays totally independent from the gRNA and PAM sequences. The bioinformatic pipeline relies on a pattern recognition algorithm, searching for specific arrangement of aligned sequence reads, i.e. reads with overlapping starts (first 3 bases) and ends (last 3 bases). Briefly, the entire genome was scanned to identify genomic regions, that were termed bins, and that contain putative cleavage sites. These sites are defined by the presence of multiple reads that either start or end at the same position (+/- 3 nt). Eventually a machine learning algorithm was introduced, based on random forest, to remove false positive cleavage sites. Selected bins were finally sorted according to their coverage (see method section and Figure 4 for details).

### Example 2: Validation of Abnoba-Seq workflow

To validate our method, Abnoba-Seq was applied to genomic DNA of the human cell line U2OS and used four different CRISPR-Cas nucleases which were previously evaluated by GUIDE-Seq and CIRCLE-Seq (Tsai et al. 2015, Tsai et al. 2017). Like expected, alignment of NGS reads to the human reference genome indicated the position of the cleavage site in the human genome with most reads starting or ending three nucleotides upstream of the PAM (Figure 5). Additional reads were observed, which did neither start nor end at the cleavage site but were part of the cluster of reads encompassing the target sites. These DNA fragments likely started or ended at the cleavage site, but were not sequenced in their complete length due to a maximum sequence read length of 150 nt.

Bioinformatical analysis revealed 42 target sites for the CRISPR-Cas9 nuclease targeting FANCF, 51 sites for the RNF2 targeting nuclease, 307 target sites for the VEGFA targeting nuclease, and 233 target sites for the CRISPR-Cas9 nuclease targeting HEK site 4 (HEKs4) (Figure 6). Of note, for Abnoba-Seq, like for GUIDE-Seq and CIRCLE-Seq, the on-target site often did not return the highest number of reads. Most identified off-target sites showed a high degree of sequence homology to the on-target site. Interestingly however, in addition to simple mispairing of nucleotides, Abnoba-Seq identified a large number of off-target sites with bulges in either the gRNA or the DNA target site.

A side-by-side comparison of the off-target sites identified by Abnoba-Seq with the off-target sites previously found by GUIDE-Seq and CIRCLE-Seq revealed a reasonable degree of overlap for the FANCF, VEGFA, and HEKs4 but not the RNF2 targeting nucleases, respectively (Figure 3). Between 58% and 100% of GUIDE-Seq and 11-21% of CIRLCE-Seq discovered off-target sites, respectively, were identified by Abnoba-Seq (Table 2). The on-target sites were identified by all three methods. On the other hand, more qualitative comparison exposed that most of the top 20 identified off-target sites were detected by all three methods (Table 2), suggesting that these three assays are able to reliably detect frequently cut off-target sites.

In summary, Abnoba-Seq was able to identify commonly cut off-target sites with substantially less genomic input DNA, making it a suitable method to evaluate target-specific programmable nuclease specificity in precious cell samples of limited sample size and availability.

### Example 3: Abnoba-Seq analysis in cell samples of limited sample size and availability

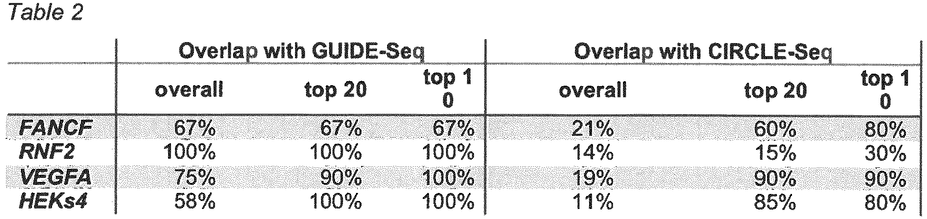

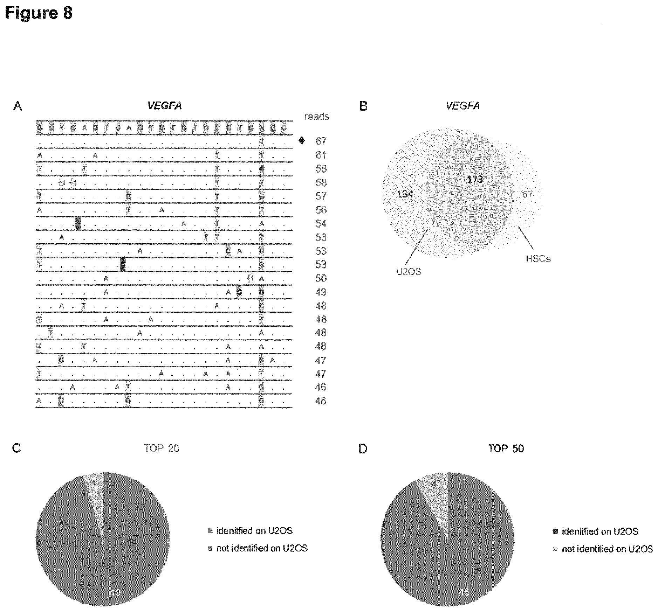

One major disadvantage of already existing off-target analysis methods is that they cannot be easily applied to cell samples of limited sample size and availability. As a proof-of-concept that Abnoba-Seq can be performed on genomic DNA of clinically relevant cell types with limited availability, Abnoba-Seq was applied to isolated genomic DNA from cord blood-derived CD34-positive hematopoietic stem and progenitor cells (HSCs). As visualized in the in-process control (Figure 3B), CRISPR-Cas9 nuclease targeting VEGFA showed high on-target cleavage activity and the Abnoba-Seq result for HSCs (Figure 8A) was comparable to the previous one preformed on genomic DNA of U2OS cells (Figure 6). A more detailed comparison revealed that a majority of the identified sites were common between the two samples (Figure 8B). When considering the top 20 or top 50 target sites identified on genomic DNA of HSCs, 95% (19/20) or 92% (46/50) were previously identified on genomic DNA of U2OS cells, respectively (Figure 8C,D), indicating high reproducibility of the assay. In summary, Abnoba-Seq is a robust method that can be successfully applied to genomic DNA isolated from precious cell samples of limited sample size and availability.

### Example 4: Verification of CRISPR-Cas off-target activity in cellula

Analysis of the *in vitro* activity of a target-specific programmable nuclease returns a worst-case-scenario because (i) *in vitro* all potential genomic off-target sites are easily accessible to CRISPR-Cas9 and (ii) the nuclease concentration might be much higher *in vitro* than in the nucleus of transfected cells. Therefore the top 20 Abnoba-Seq predicted off-target sites were analysed for the VEGFA and HEKs4 targeting CIRPSR-Cas nucleases by targeted amplicon sequencing seven days after transfection of U2OS cells or primary HSCs. The overall editing efficiency, as indicated by the indel frequencies, was 96% at the VEGFA on-target site in U2OS cells, 61% at the VEGFA on-target site in HSCs, and 69% at the HEKs4 on-target site in HSCs (Figure 9). The overall off-target activity in these cells was rather low. The highest off-target activity was observed at VEGFA off-target site (OT) 8, with 20% edited alleles in U2OS cells and 10% edited alleles in HSCs, and VEGFA OT-13 with 5% edited alleles in HSCs. Off-target activity at the other predicted off-target sites was low or absent. Some of the off-target sites were only cleaved in U2OS cells but not in primary HSCs (OT-12, OT-14, OT-16), suggesting cell type specific effects. In summary, only about half of the *in vitro* identified off-target sites were actually cleaved in cells. Of note, most of these investigated sites were also predicted to be off-target sites by CIRLCE-Seq and GUIDE-Seq. Taken together, it is important to keep in mind that *in vitro* methods to detect off-target sites in a genome-wide and unbiased manner are sophisticated but still mere prediction tools.

While many genome-wide methods to identify CRISPR-Cas associated off-target activity are seemingly unbiased, the bioinformatics pipeline to analyse the NGS data does introduce some bias, in particular with regard to the maximal number of allowed mismatches or the requirement of a PAM. Importantly, Abnoba-Seq to identify off-target sites does not make any assumptions about the nature of an off-target site, neither the degree of homology to the on-target site nor the presence of a specific PAM. Hence, Abnoba-Seq will identify off-target sites that are excluded by other methods, including RNF2 OT7 with 7 mismatches and 2 bulges, RNF2 OT37 with 9 mismatches, VEGFA OT2 with 3 mismatches and a TGA PAM, VEGFA OT26 with 3 mismatches and a TGA PAM, or VEGFA OT75 with 2 mismatches and a TTG PAM. Taken together, Abnoba-Seq is a truly unbiased *in vitro* method to profile CRISPR-Cas off-target activity *in vitro* and *in vivo.* Since only minimal amounts of genomic DNA are required to perform the assay, Abnoba-Seq can be applied in a cell type-specific manner to precious cell samples of limited sample size and availability.

## Claims

1. A method for detecting off-target sites of one or more target-specific programmable nucleases in a genome *in vitro* comprising the steps of:
(a) providing an input sample comprising genomic DNA, randomly fragmenting the genomic DNA into dsDNA fragments, and ligating a blocking adapter to the ends of the dsDNA fragments and removing dsDNA fragments comprising ends, which are not end-blocked, to prepare purified end-blocked dsDNA fragments;
(b) preparing a cleaved and amplified DNA library of dsDNA fragments, by
b1) cleaving the purified end-blocked dsDNA fragments with one or more target-specific programmable nucleases to obtain cleaved dsDNA fragments;
b2) ligating an affinity adapter to cleavage sites of cleaved dsDNA fragments to obtain affinity-adapter-modified dsDNA fragments;
b3) enriching for affinity-adapter-modified dsDNA fragments using the affinity adapter to obtain enriched affinity-adapter-modified dsDNA fragments; and
b4) amplifying enriched affinity-adapter-modified dsDNA fragments by PCR amplification to obtain a cleaved and amplified DNA library of dsDNA fragments;
(c) performing a sequencing of the cleaved and amplified DNA library of dsDNA fragments to obtain sequence reads;
(d) aligning the sequence reads to a reference genome to obtain aligned sequence reads;
(e) identifying cleavage sites, by determining putative cleavage sites and excluding false positive cleavage sites, wherein putative cleavage sites are determined by locating sequence regions in the reference genome where both sequence read starts and sequence read ends of aligned sequence reads coincide, and identifying putative cleavage sites as the sites in these sequence regions, where sequence read starts and sequence read ends meet.

2. The method according to claim 1, wherein the input sample comprises less than 1 µg, preferably less than 800 ng, more preferably less than 700 ng, even more preferably about 600 ng, of genomic DNA.

3. The method according to any of the preceding claims, wherein step a) comprises
a1) randomly fragmenting the genomic DNA to a defined length to provide dsDNA fragments;
a2) protecting the ends of the dsDNA fragments by ligating a blocking adapter to obtain end-blocked dsDNA fragments, preferably comprising the steps of
1) preparing the dsDNA fragments for blocking adapter ligation; and
2) ligating a blocking adapter to ends of dsDNA fragments, to obtain end-blocked dsDNA fragments; and
a3) contacting the sample comprising the end-blocked dsDNA fragments with one or more exonucleases to remove dsDNA fragments, which are not end-blocked, to obtain purified end-blocked DNA fragments.

4. The method according to any of the preceding claims, wherein the blocking adapter comprises one or more modifications, which after ligation of the blocking adapter to a dsDNA fragment, prevent ligation, of the end to which the blocking adapter is ligated to the dsDNA fragment and one or more of modifications, which after ligation of the blocking adapter to a dsDNA fragment, prevent exonuclease cleavage of the dsDNA fragment from the end to which the blocking adapter is ligated to the dsDNA fragment.

5. The method according to any of the preceding claims, wherein in step b1) the one or more target-specific programmable nucleases is selected from Transcription Activator-Like Effector Nucleases (TALEN), zinc finger nucleases (ZFN), meganucleases, megaTAL, Fokl-dCas9, Clustered Regularly Interspaced Short Palindromic Repeats (CRISPR)/Cas RNA-guided nucleases (CRISPR/Cas RGN) or a variant thereof, Argonaute-family protein-based DNA-guided DNA nucleases or a variant thereof, or a combination thereof, preferably one or more CRISPR/Cas RGN, wherein the Cas endonuclease of the CRISPR/Cas RGN is preferably Cas9 endonuclease or a variant of Cas9 endonuclease or Cpf1 endonuclease or a variant of Cpf1 endonuclease.

6. The method according to any of the preceding claims, wherein the blocking adapter and/or the affinity adapter are dsDNA-based adapters, and wherein the blocking adapter and/or the affinity adapter comprise a primer site compatible for use in PCR priming.

7. The method according to any of the preceding claims, wherein the affinity adapter is a dsDNA-based adapter comprising one or more biotin or biotin-based modifications and wherein in step b3) streptavidin or a variant thereof, preferably immobilized on magnetic beads, is used as an affinity adapter binding molecule to enrich for cleaved and affinity-adapter-modified dsDNA fragments.

8. The method according to any of the preceding claims, wherein step b4) to obtain the cleaved and amplified DNA library of dsDNA fragments comprises a two-step PCR amplification, with
1. a first PCR amplification step for linear PCR amplification using enriched affinity-adapter-modified dsDNA fragments bound via an adapter binding molecule to a solid support as a template and an affinity-adapter-specific primer; and
2. after the first step a second PCR amplification step, after removal of enriched affinity adapter modified dsDNA fragments bound via adapter binding molecule to the solid support from the reaction mix and addition of a blocking-adapter-specific primer as a second primer.

9. The method according to any of the preceding claims, wherein in step d) the sequence reads are aligned to a reference genome to obtain aligned sequence reads, by first
d1) trimming off blocking adapter, affinity adapter, and optional sequencing adapter sequence from sequence reads to obtain trimmed sequence reads; and second
d2) mapping the trimmed sequence reads to a reference genome to obtain aligned sequence reads.

10. The method according to any of the preceding claims, wherein the genomic DNA is human genomic DNA and wherein the reference genome is a human reference genome, preferably hg19 or hg38.

11. The method according to any of the preceding claims, wherein in step e) putative cleavage sites are determined by
- dividing the reference genome comprising aligned sequence reads into bins of a defined length, preferably of at least about 100 nt; and
- determining for each bin comprising at least one sequence read start and at least one sequence read end, one or more putative cleavage sites as sites with a local maximum for the sum of sequence read starts and sequence read ends, and optionally minimizing the distance to the centre of the bin;
wherein a sequence read start consists of the first 1 to 5 nt, preferably the first 2 to 4 nt, more preferably the first 3 nt, and a sequence read end consists of the last 1 to 5 nt, preferably the last 2 to 4 nt, more preferably the last 3 nt, of an aligned sequence read.

12. The method according to any of the preceding claims, wherein in step e) cleavage sites are identified by
e1) dividing the reference genome comprising aligned sequence reads into bins of set sequence length, preferably of about 100 nt length,
e2) preferably determining for each bin a read coverage, discarding bins with a read coverage of 0 and preferably combining bins with a read coverage of >0 consecutively, based on the sequence order in the reference genome, into a continuous set of bins;
e3) dividing each bin into windows of set width, of preferably 4 nt, with a set step size, which is equal or smaller, preferably smaller, than the width of the windows, of preferably 2 nt;
e4) determining start, end and background coverage of each window, by grouping,
- sequence read starts falling in the window region into a start coverage group;
- sequence read ends falling in the window region into an end coverage group; and
- sequence reads falling into the window region but not belonging to the start coverage group and the end coverage group into a background coverage group;
and summing up for each window the number of reads in the start coverage, end coverage and background coverage group, respectively;
e5) determining for each bin comprising at least one sequence read start and at least one sequence read end, one or more putative cleavage sites by maximizing for the sum of start coverage and end coverage and minimizing the distance to the centre of the bin; and
e6) excluding false positive cleavage sites;
wherein a sequence read start consists of the first 1 to 5 nt, preferably the first 2 to 4 nt, more preferably the first 3 nt, and a sequence read end consists of the last 1 to 5 nt, preferably the last 2 to 4 nt, more preferably the last 3 nt, of an aligned sequence read.

13. The method according to any of the preceding claims, wherein false positive cleavage sites are excluded by selecting only putative cleavage sites with
1. a ratio of background coverage to signal coverage, which does not exceed a certain threshold, preferably about 0.5; and/or
2. a ratio of start coverage to end coverage at the putative cleavage site, falling within a certain numerical range, preferably of between about 1/5 and 5/1; and/or
3. a minimum site coverage, of preferably 6 or more; and/or
4. a minimum bin coverage, of preferably 10 or more.

14. A method for detecting off-target sites in a genome *in vivo* comprising the steps of
i) providing a first sample, comprising genomic DNA of cells treated with one or more target-specific programmable nucleases, and a second sample, comprising genomic DNA of cells of related genetic background, preferably cells of the same genetic background, not treated with the one or more target-specific programmable nucleases;
ii) for both samples independently detecting off-target sites *in vitro* by using the method of claims 1 to 13 and using the one or more target-specific programmable nucleases of step i); and
iii) comparing off-target sites between the two samples to identify off-target sites only identified in the sample comprising genomic DNA of cells not treated with the one or more target-specific programmable nucleases, but not in the sample comprising genomic DNA of cells treated with the one or more target-specific programmable nucleases as *in vivo* off-target sites of the one or more target-specific programmable nucleases.

## Patentansprüche

1. Verfahren zum Nachweisen von Off-Target-Stellen einer oder mehrerer zielspezifischer programmierbarer Nukleasen in einem Genom *in vitro,* umfassend die folgenden Schritte:
(a) Bereitstellen einer Einsatzprobe, die genomische DNA umfasst, zufälliges Fragmentieren der genomischen DNA in dsDNA-Fragmente und Ligieren eines Blockierungsadapters an die Enden der dsDNA-Fragmente und Entfernen der dsDNA-Fragmente umfassenden Enden, die nicht end-blockiert sind, wodurch gereinigte endblockierte dsDNA-Fragmente erzeugt werden;
(b) Erstellen einer gespaltenen und amplifizierten DNA-Bibliothek von dsDNA-Fragmenten durch
b1) Spalten der gereinigten end-blockierten dsDNA-Fragmente mit einer oder mehreren zielspezifischen programmierbaren Nukleasen unter Erhalt gespaltener dsDNA-Fragmente;
b2) Ligieren eines Affinitätsadapters an Spaltstellen von gespaltenen dsDNA-Fragmenten unter Erhalt von Affinitätsadapter-modifizierten dsDNA-Fragmenten;
b3) Anreichern Affinitätsadapter-modifizierter dsDNA-Fragmente unter Verwendung des Affinitätsadapters unter Erhalt angereicherter Affinitätsadapter-modifizierter dsDNA-Fragmente und
b4) Amplifizieren angereicherter Affinitätsadapter-modifizierter dsDNA-Fragmente durch PCR-Amplifikation unter Erhalt einer gespaltenen und amplifizierten DNA-Bibliothek von dsDNA-Fragmenten;
(c) Durchführen einer Sequenzierung der gespaltenen und amplifizierten DNA-Bibliothek von dsDNA-Fragmenten unter Erhalt von Sequenzlesungen;
(d) Anordnen der Sequenzlesungen in Bezug auf ein Referenzgenom unter Erhalt angeordneter Sequenzlesungen;
(e) Identifizieren von Spaltstellen durch Bestimmen mutmaßlicher Spaltstellen und Ausschließen falsch positiver Spaltstellen, wobei mutmaßliche Spaltstellen durch Lokalisierung von Sequenzregionen in dem Referenzgenom, in denen sowohl Sequenzlesungs-Anfänge als auch Sequenzlesungs-Enden angeordneter Sequenzlesungen zusammenfallen, bestimmt werden, und Identifizieren mutmaßlicher Spaltstellen als die Stellen in diesen Sequenzregionen, an denen sich Sequenzlesungs-Anfänge und Sequenzlesungs-Enden treffen.

2. Verfahren nach Anspruch 1, wobei die Einsatzprobe weniger als 1 µg, vorzugsweise weniger als 800 ng, stärker bevorzugt weniger als 700 ng, noch stärker bevorzugt etwa 600 ng von genomischer DNA umfasst.

3. Verfahren nach einem der vorstehenden Ansprüche, wobei Schritt a)
a1) zufälliges Fragmentieren der genomischen DNA auf eine definierte Länge, um dsDNA-Fragmente bereitzustellen;
a2) Schützen der Enden der dsDNA-Fragmente durch Ligieren eines Blockierungsadapters unter Erhalt von end-blockierten dsDNA-Fragmenten, vorzugsweise umfassend die Schritte
1) Herstellen der dsDNA-Fragmente für Blockierungsadapter-Ligation und
2) Ligieren eines Blockierungsadapters an Enden von dsDNA-Fragmenten unter Erhalt von end-blockierten dsDNA-Fragmenten; und
a3) Kontaktieren der die end-blockierten dsDNA-Fragmente umfassenden Probe mit einer oder mehreren Exonukleasen zum Entfernen von dsDNA-Fragmenten, die nicht end-blockiert sind, unter Erhalt von gereinigten end-blockierten DNA-Fragmenten
umfasst.

4. Verfahren nach einem der vorstehenden Ansprüche, wobei der Blockierungsadapter eine oder mehrere Modifikationen, die nach Ligation des Blockierungsadapters an ein dsDNA-Fragment Ligation des Endes, an dem der Blockierungsadapter an das dsDNA-Fragment ligiert ist, verhindern, und eine oder mehrere Modifikationen, die nach Ligation des Blockierungsadapters an ein dsDNA-Fragment Exonuklease-Spaltung des dsDNA-Fragments von dem Ende, an dem der Blockierungsadapter an das dsDNA-Fragment ligiert ist, verhindern, umfasst.

5. Verfahren nach einem der vorstehenden Ansprüche, wobei in Schritt b1) die eine oder mehreren zielspezifischen programmierbaren Nukleasen aus Transkriptionsaktivator-ähnlichen Effektornukleasen (TALEN), Zinkfingernukleasen (ZFN), Meganukleasen, megaTAL, Fokl-dCas9, Clustered Regularly Interspaced Short Palindromic Repeats (CRISPR)/Cas RNA-geführte Nukleasen (CRISPR/Cas RGN) oder einer Variante davon, Argonautenproteinbasierten DNA-geführten DNA-Nukleasen oder einer Variante davon oder einer Kombination davon, vorzugsweise einer oder mehreren CRISPR/Cas RGN, ausgewählt sind, wobei die Cas-Endonuklease der CRISPR/Cas RGN vorzugsweise Cas9-Endonuklease oder eine Variante von Cas9-Endonuklease oder Cpf1-Endonuklease oder eine Variante von Cpf1-Endonuklease ist.

6. Verfahren nach einem der vorstehenden Ansprüche, wobei der Blockierungsadapter und/oder der Affinitätsadapter dsDNA-basierte Adapter sind/ist und wobei der Blockierungsadapter und/oder der Affinitätsadapter eine Primer-Stelle umfassen/umfasst, die zur Verwendung beim PCR-Priming kompatibel ist.

7. Verfahren nach einem der vorstehenden Ansprüche, wobei der Affinitätsadapter ein dsDNA-basierter Adapter ist, der eine oder mehrere Biotin- oder Biotin-basierte Modifikationen umfasst, und wobei in Schritt b3) Streptavidin oder eine Variante davon, das vorzugsweise auf Magnetkügelchen immobilisiert ist, als ein Affinitätsadapterbindungsmolekül verwendet wird, um gespaltene und Affinitätsadapter-modifizierte dsDNA-Fragmente anzureichern.

8. Verfahren nach einem der vorstehenden Ansprüche, wobei Schritt b4) zum Erhalt der gespaltenen und amplifizierten DNA-Bibliothek von dsDNA-Fragmenten eine Zweischritt-PCR-Amplifikation mit
1. einem ersten PCR-Amplifikationsschritt für lineare PCR-Amplifikation unter Verwendung angereicherter Affinitätsadapter-modifizierter dsDNA-Fragmente, die über ein Adapterbindungsmolekül an einen festen Träger gebunden sind, als eine Matrize und eines Affinitätsadapter-spezifischen Primers und
2. nach dem ersten Schritt einem zweiten PCR-Amplifikationsschritt nach der Entfernung von angereicherten Affinitätsadapter-modifizierten dsDNA-Fragmenten, die über ein Adapterbindungsmolekül an den festen Träger gebunden sind, aus der Reaktionsmischung und Zugabe eines Blockierungsadapter-spezifischen Primers als ein zweiter Primer
umfasst.

9. Verfahren nach einem der vorstehenden Ansprüche, wobei in Schritt d) die Sequenzlesungen in Bezug auf ein Referenzgenom unter Erhalt angeordneter Sequenzlesungen durch erstens
d1) Zuschneiden einer Blockierungsadapter-, Affinitätsadapter- und optionalen Sequenzierungsadaptersequenz von Sequenzlesungen unter Erhalt zugeschnittener Sequenzlesungen und zweitens
d2) Kartieren der zugeschnittenen Sequenzlesungen in Bezug auf ein Referenzgenom unter Erhalt angeordneter Sequenzlesungen
angeordnet werden.

10. Verfahren nach einem der vorstehenden Ansprüche, wobei die genomische DNA humane genomische DNA ist und wobei das Referenzgenom ein humanes Referenzgenom, vorzugsweise hg19 oder hg38 ist.

11. Verfahren nach einem der vorstehenden Ansprüche, wobei in Schritt e) mutmaßliche Spaltstellen bestimmt werden durch
- Teilen des Referenzgenoms, das angeordnete Sequenzlesungen umfasst, in Bins mit einer definierten Länge, vorzugsweise mindestens etwa 100 nt; und
- Bestimmen für jedes Bin, das mindestens einen Sequenzlesungs-Anfang und mindestsens ein Sequenzlesungs-Ende umfasst, einer oder mehrerer mutmaßlicher Spaltstellen als Stellen mit einem lokalen Maximum für die Summe von Sequenzlesungs-Anfängen und Sequenzlesungs-Enden und gegebenenfalls Minimieren des Abstands zum Zentrum des Bins;
wobei ein Sequenzlesungs-Anfang aus den ersten 1 bis 5 nt, vorzugsweise den ersten 2 bis 4 nt, stärker bevorzugt den ersten 3 nt, und ein Sequenzlesungs-Ende aus den letzten 1 bis 5 nt, vorzugsweise den letzten 2 bis 4 nt, stärker bevorzugt den letzten 3 nt, einer angeordneten Sequenzlesung besteht.

12. Verfahren nach einem der vorstehenden Ansprüche, wobei in Schritt e) Spaltstellen identifiziert werden durch
e1) Teilen des Referenzgenoms, das angeordnete Sequenzlesungen umfasst, in Bins mit einer festgelegten Sequenzlänge, vorzugsweise einer Länge von etwa 100 nt,
e2) vorzugsweise Bestimmen einer Leseabdeckung für jedes Bin, Verwerfen von Bins mit einer Leseabdeckung von 0 und vorzugsweise fortlaufendes Vereinigen von Bins mit einer Leseabdeckung von >0, basierend auf der Sequenzreihenfolge in dem Referenzgenom, in einem kontinuierlichen Satz von Bins;
e3) Teilen jedes Bins in Fenster mit einer Satzbreite von vorzugsweise 4 nt mit einer Satzschrittgröße, die gleich oder kleiner, vorzugsweise kleiner ist als die Breite der Fenster, von vorzugsweise 2 nt;
e4) Bestimmen der Anfangs-, End- und Hintergrundabdeckung jedes Fensters durch Gruppieren von
- Sequenzlesungs-Anfängen, die in die Fensterregion fallen, in eine Anfangsabdeckungsgruppe;
- Sequenzlesungs-Enden, die in die Fensterregion fallen, in eine Endabdeckungsgruppe und
- Sequenzlesungen, die in die Fensterregion fallen, aber nicht zur der Anfangsabdeckungsgruppe und der Endabdeckungsgruppe gehören, in eine Hintergrundabdeckungsgruppe;
und Summieren der Lesungen in der Anfangsabdeckungs-, der Endabdeckungs- bzw. der Hintergrundabdeckungsgruppe für jedes Fenster;
e5) Bestimmen für jedes Bin, das mindestens einen Sequenzlesungs-Anfang und mindestens ein Sequenzlesungs-Ende umfasst, einer oder mehrerer mutmaßlicher Spaltstellen durch Maximieren der Summe von Anfangsabdeckung und Endabdeckung und Minimieren des Abstands zum Zentrum des Bins und
e6) Ausschließen falsch positiver Spaltstellen;
wobei ein Sequenzlesungs-Anfang aus den ersten 1 bis 5 nt, vorzugsweise den ersten 2 bis 4 nt, stärker bevorzugt den ersten 3 nt, und ein Sequenzlesungs-Ende aus den letzten 1 bis 5 nt, vorzugsweise den letzten 2 bis 4 nt, stärker bevorzugt den letzten 3 nt, einer angeordneten Sequenzlesung besteht.

13. Verfahren nach einem der vorstehenden Ansprüche, wobei falsch positive Spaltstellen durch Auswählen lediglich mutmaßlicher Spaltstellen mit
1. einem Verhältnis von Hintergrundabdeckung zu Signalabdeckung, das einen bestimmten Schwellenwert nicht übersteigt, von vorzugsweise etwa 0,5 und/oder
2. einem Verhältnis von Anfangsabdeckung zu Endabdeckung an der mutmaßlichen Spaltstelle, das in einen bestimmten Zahlenbereich fällt, vorzugsweise von zwischen etwa 1/5 und 5/1 und/oder
3. einer minimalen Stellenabdeckung von vorzugsweise 6 oder mehr und/oder
4. einer minimalen Bin-Abdeckung von vorzugsweise 10 oder mehr
ausgeschlossen werden.

14. Verfahren zum Nachweisen von Off-Target-Stellen in einem Genom *in vivo,* umfassend die Schritte
i) Bereitstellen einer ersten Probe, die genomische DNA von Zellen umfasst, die mit einer oder mehreren zielspezifischen programmierbaren Nukleasen behandelt wurden, und einer zweiten Probe, die genomische DNA von Zellen mit verwandtem genetischem Hintergrund, vorzugsweise Zellen mit demselben genetischen Hintergrund, die nicht mit einer oder mehreren zielspezifischen programmierbaren Nukleasen behandelt wurden, umfasst;
ii) nachweisen von Off-Target-Stellen *in vitro* für beide Proben unabhängig unter Anwendung des Verfahrens nach den Ansprüchen 1 bis 13 und unter Verwendung der einen oder mehreren zielspezifischen programmierbaren Nukleasen von Schritt i) und
iii) Vergleichen der Off-Target-Stellen zwischen den beiden Proben zum Identifizieren von Off-Target-Stellen, die nur in der Probe identifiziert wurden, die genomische DNA von Zellen umfasst, die nicht mit einer oder mehreren zielspezifischen programmierbaren Nukleasen behandelt wurden, nicht aber in der Probe identifiziert wurden, die genomische DNA von Zellen umfasst, die mit einer oder mehreren zielspezifischen programmierbaren Nukleasen behandelt wurden, als *In*-*vivo*-Off-Target-Stellen der einen oder mehreren zielspezifischen programmierbaren Nukleasen.

## Revendications

1. Procédé de détection de sites hors cible d'une ou plusieurs nucléases programmables spécifiques à une cible dans un génome in vitro comprenant les étapes consistant à :
(a) fournir un échantillon d'entrée comprenant de l'ADN génomique, fragmentation aléatoire de l'ADN génomique en fragments d'ADNdb, et ligaturer un adaptateur de blocage aux extrémités des fragments d'ADNdb et éliminer des fragments d'ADNdb comprenant des extrémités, qui ne sont pas à extrémité bloquée, pour préparer des fragments d'ADNdb à extrémité bloquée purifiés;
(b) préparer une bibliothèque d'ADN clivé et amplifié de fragments d'ADNdb, consistant à
b1) cliver les fragments d'ADNdb à extrémité bloquée purifiés avec une ou plusieurs nucléases programmables spécifiques de la cible pour obtenir des fragments d'ADNdb clivés ;
b2) ligaturer un adaptateur d'affinité aux sites de clivage des fragments d'ADNdb clivés pour obtenir des fragments d'ADNdb modifiés par un adaptateur d'affinité ;
b3) enrichir les fragments d'ADNdb modifiés par l'adaptateur d'affinité en utilisant l'adaptateur d'affinité pour obtenir des fragments d'ADNdb modifiés par l'adaptateur d'affinité enrichis ; et
b4) amplifier les fragments d'ADNdb modifiés par l'adaptateur d'affinité enrichis par amplification PCR pour obtenir une bibliothèque d'ADN de fragments d'ADNdb clivés et amplifiés ;
(c) réaliser un séquençage de la bibliothèque d'ADN clivé et amplifié de fragments d'ADNdb pour obtenir des lectures de séquence ;
(d) aligner les lectures de séquence sur un génome de référence pour obtenir des lectures de séquence alignées ;
(e) identifier les sites de clivage, en déterminant les sites de clivage putatifs et en excluant les sites de clivage faussement positifs, dans lequel les sites de clivage putatifs sont déterminés en localisant les régions de séquence dans le génome de référence où les débuts de lecture de séquence et les fins de lecture de séquence des lectures de séquence alignées coïncident, et identifier les sites de clivage putatifs comme les sites dans ces régions de séquence, où les débuts de lecture de séquence et les fins de lecture de séquence se rencontrent.

2. Procédé selon la revendication 1, dans lequel l'échantillon d'entrée comprend moins de 1 µg, de préférence moins de 800 ng, plus préférablement moins de 700 ng, encore plus préférablement environ 600 ng, d'ADN génomique.

3. Procédé selon l'une quelconque des revendications précédentes, dans lequel l'étape a) consiste à
a1) fragmenter de façon aléatoire l'ADN génomique à une longueur définie pour fournir des fragments d'ADNdb ;
a2) protéger les extrémités des fragments d'ADNdb par ligature d'un adaptateur de blocage pour obtenir des fragments d'ADNdb à extrémité bloquée, comprenant de préférence les étapes consistant à
1) préparer les fragments d'ADNdb pour la ligature d'un adaptateur de blocage ; et
2) ligaturer un adaptateur de blocage aux extrémités des fragments d'ADNdb, pour obtenir des fragments d'ADNdb à extrémité bloquée ; et
a3) mettre en contact l'échantillon comprenant les fragments d'ADNdb à extrémité bloquée avec une ou plusieurs exonucléases pour éliminer les fragments d'ADNdb qui ne sont pas à extrémité bloquée, pour obtenir des fragments d'ADN à extrémité bloquée purifiés.

4. Procédé selon l'une quelconque des revendications précédentes, dans lequel l'adaptateur de blocage comprend une ou plusieurs modifications, qui après ligature de l'adaptateur de blocage à un fragment d'ADNdb, empêchent la ligature de l'extrémité à laquelle l'adaptateur de blocage est ligaturé au fragment d'ADNdb et une ou plusieurs modifications, qui après ligature de l'adaptateur de blocage à un fragment d'ADNdb, empêchent le clivage par exonucléase du fragment d'ADNdb à partir de l'extrémité à laquelle l'adaptateur de blocage est ligaturé au fragment d'ADNdb.

5. Procédé selon l'une quelconque des revendications précédentes, dans lequel, à l'étape b1), la ou les nucléases programmables spécifiques à une cible sont choisies parmi les nucléases effectrices de type activateur de transcription (TALEN), les nucléases à doigt de zinc (ZFN), les méganucléases, megaTAL, FokI-dCas9, les répétitions palindromiques courtes régulièrement espacées (CRISPR)/Cas guidées par l'ARN (CRISPR/Cas RGN) ou une variante de celles-ci, nucléases ADN guidées par l'ADN à base de protéines de la famille des argonautes ou une variante de celles-ci, ou une combinaison de celles-ci, de préférence un ou plusieurs CRISPR/Cas RGN, où l'endonucléase Cas du CRISPR/Cas RGN est de préférence l'endonucléase Cas9 ou une variante de l'endonucléase Cas9 ou l'endonucléase Cpf1 ou une variante de l'endonucléase Cpf1.

6. Procédé selon l'une quelconque des revendications précédentes, dans lequel l'adaptateur de blocage et/ou l'adaptateur d'affinité sont des adaptateurs à base d'ADNdb, et dans lequel l'adaptateur de blocage et/ou l'adaptateur d'affinité comprennent un site d'amorce compatible pour une utilisation dans l'amorçage PCR.

7. Procédé selon l'une quelconque des revendications précédentes, dans lequel l'adaptateur d'affinité est un adaptateur à base d'ADNdb comprenant une ou plusieurs biotine ou modifications à base de biotine et dans lequel, à l'étape b3), la streptavidine ou une variante de celle-ci, de préférence immobilisée sur des billes magnétiques, est utilisée comme molécule de liaison d'adaptateur d'affinité pour enrichir les fragments d'ADNdb clivés et modifiés par l'adaptateur d'affinité.

8. Procédé selon l'une quelconque des revendications précédentes, dans lequel l'étape b4) pour obtenir la bibliothèque d'ADN clivé et amplifié de fragments d'ADNdb comprend une amplification PCR en deux étapes, avec
1. une première étape d'amplification PCR pour une amplification PCR linéaire utilisant des fragments d'ADNdb enrichis modifiés par affinité et adaptateur, liés via une molécule de liaison d'adaptateur à un support solide comme matrice et une amorce spécifique d'adaptateur d'affinité ; et
2. après la première étape, une seconde étape d'amplification PCR, après élimination du mélange réactionnel des fragments d'ADNdb modifiés par l'adaptateur d'affinité enrichi lié via la molécule de liaison de l'adaptateur au support solide et addition d'une amorce spécifique d'adaptateur bloquant comme une seconde amorce.

9. Procédé selon l'une quelconque des revendications précédentes, dans lequel, à l'étape d), les lectures de séquence sont alignées sur un génome de référence pour obtenir des lectures de séquence alignées, en effectuant premièrement à
d1) découper l'adaptateur de blocage, l'adaptateur d'affinité et la séquence d'adaptateur de séquençage facultatif des lectures de séquence pour obtenir des lectures de séquence découpées ; et deuxièmement à
d2) mettre en correspondance des lectures de séquence découpées avec un génome de référence pour obtenir des lectures de séquence alignées.

10. Procédé selon l'une quelconque des revendications précédentes, dans lequel l'ADN génomique est un ADN génomique humain et dans lequel le génome de référence est un génome de référence humain, de préférence hg19 ou hg38.

11. Procédé selon l'une quelconque des revendications précédentes, dans lequel, à l'étape e), des sites de clivage putatifs sont déterminés par les étapes consistant à
- diviser le génome de référence comprenant des lectures de séquences alignées en bacs d'une longueur définie, de préférence d'au moins environ 100 nt ; et
- déterminer pour chaque bac comprenant au moins un début de lecture de séquence et au moins une fin de lecture de séquence, un ou plusieurs sites de clivage putatifs en tant que sites avec un maximum local pour la somme des débuts de lecture de séquence et des fins de lecture de séquence, et éventuellement en minimisant la distance au centre du bac ;
dans lequel un début de lecture de séquence consiste en le premier 1 à 5 nt, de préférence le premier 2 à 4 nt, plus préférablement le premier 3 nt, et une fin de lecture de séquence consiste en le dernier 1 à 5 nt, de préférence le dernier 2 à 4 nt, plus préférablement le dernier 3 nt, d'une lecture de séquence alignée.

12. Procédé selon l'une quelconque des revendications précédentes, dans lequel, à l'étape e), les sites de clivage sont identifiés par les étapes consistant à
e1) diviser le génome de référence comprenant des lectures de séquences alignées en bacs d'ensemble de longueur de séquence, de préférence d'une longueur d'environ 100 nt,
e2) déterminer de préférence pour chaque bac une couverture de lecture, en écartant les bacs avec une couverture de lecture de 0 et en combinant de préférence les bacs avec une couverture de lecture de >0 consécutivement, sur la base de l'ordre de séquence dans le génome de référence, en un ensemble continu de bacs ;
e3) diviser chaque bac en fenêtres de largeur définie, de préférence 4 nt, avec une taille de pas définie, qui est égale ou inférieure, de préférence inférieure, à la largeur des fenêtres, de préférence 2 nt ;
e4) déterminer la couverture de début, de fin et de fond de chaque fenêtre, en regroupant
- les débuts de lecture de séquences tombant dans la région de fenêtre dans un groupe de couverture de début ;
- les fins de lecture de séquences tombant dans la région de fenêtre dans un groupe de couverture de fin ; et
- les lectures de séquence tombant dans la région de fenêtre mais n'appartenant pas au groupe de couverture de début et au groupe de couverture de fin dans un groupe de couverture de fond ;
et additionner pour chaque fenêtre le nombre de lectures dans le groupe de couverture de début, le groupe de couverture de fin et le groupe de couverture de fond, respectivement ;
e5) déterminer pour chaque bac comprenant au moins un début de lecture de séquence et au moins une fin de lecture de séquence, un ou plusieurs sites de clivage putatifs en maximisant la somme de la couverture de début et de la couverture de fin et en minimisant la distance au centre du bac ; et
e6) exclure les sites de clivage faussement positifs ;
dans lequel un début de lecture de séquence consiste en le premier 1 à 5 nt, de préférence le premier 2 à 4 nt, plus préférablement le premier 3 nt, et une fin de lecture de séquence consiste en le dernier 1 à 5 nt, de préférence le dernier 2 à 4 nt, plus préférablement le dernier 3 nt, d'une lecture de séquence alignée.

13. Procédé selon l'une quelconque des revendications précédentes, dans lequel les sites de clivage faussement positifs sont exclus en sélectionnant uniquement les sites de clivage putatifs ayant
1. un rapport entre la couverture de fond et la couverture de signal, qui ne dépasse pas un certain seuil, de préférence environ 0,5 ; et/ou
2. un rapport entre la couverture de début et la couverture de fin au niveau du site de clivage putatif, compris dans une certaine plage numérique, de préférence entre environ 1/5 et 5/1 ; et/ou
3. une couverture minimale de site, de préférence de 6 ou plus ; et/ou
4. une couverture minimale de bac, de préférence de 10 ou plus.

14. Procédé de détection de sites hors cible dans un génome *in vivo* comprenant les étapes consistant à
i) fournir un premier échantillon, comprenant l'ADN génomique de cellules traitées avec une ou plusieurs nucléases programmables spécifiques à une cible, et un second échantillon, comprenant l'ADN génomique de cellules de fond génétique apparenté, de préférence des cellules de même fond génétique, non traitées avec la ou les nucléases programmables spécifiques à une cible ;
ii) pour les deux échantillons, détecter indépendamment les sites hors cible *in vitro* en utilisant le procédé des revendications 1 à 13 et en utilisant la ou les nucléases programmables spécifiques à une cible de l'étape i) ; et
iii) comparer les sites hors cible entre les deux échantillons pour identifier les sites hors cible identifiés uniquement dans l'échantillon comprenant l'ADN génomique de cellules non traitées avec une ou plusieurs nucléases programmables spécifiques à une cible, mais pas dans l'échantillon comprenant l'ADN génomique de cellules traitées avec une ou plusieurs nucléases programmables spécifiques à une cible comme sites hors cible in vivo de la ou des nucléases programmables spécifiques à une cible.
